Europäisches Patentamt
European Patent Office
Office européen des brevets

(11) Publication number: 0 290 203
A1

# EUROPEAN PATENT APPLICATION

(21) Application number: 88303869.7

(22) Date of filing: 28.04.88

(51) Int. Cl.⁴: C07H 17/08 , A61K 31/70

(30) Priority: 29.04.87 US 44076

(43) Date of publication of application:
09.11.88 Bulletin 88/45

(84) Designated Contracting States:
ES GR

(71) Applicant: SCHERING CORPORATION
2000 Galloping Hill Road
Kenilworth, New Jersey 07033(US)

(72) Inventor: Mallams, Alan K.
147 Kings Highway
Hackettstown New Jersey 07840(US)
Inventor: Sarre, Olga Z.
12 Valhalla Way
Verona New Jersey 07044(US)
Inventor: Girijavallabhan, Viyyoor M.
10 Maplewood Drive
Parsippany New Jersey 07054(US)
Inventor: Rossman, Randall Robert
82 Oakley Terrace
Nutley New Jersey 07110(US)
Inventor: Ganguly, Ashit Kumar
96 Cooper Avenue
Upper Montclair New Jersey 07043(US)

(74) Representative: Ritter, Stephen David et al
Mathys & Squire 10 Fleet Street
London EC4Y 1AY(GB)

## (54) 3-O-glycosyl 16-membered macrolide antibacterials and related derivatives.

(57) 3-O-Glycosyl derivatives of 16-membered macrolides such as O-α-L-cladinosyl-(1→3)-12,13-dehydro-12,13-deoxorosaramicin, 2″,4″,4‴-tri-O-acetyl-O-(4′-O-phenoxyacetyl-α-L-cladinosyl)-(1→3)-desmycosin and pharmaceutically acceptable acid addition salts thereof useful as antibacterials are disclosed.

EP 0 290 203 A1

## 3-O-GLYCOSYL 16-MEMBERED MACROLIDE ANTIBACTERIALS AND RELATED DERIVATIVES

This invention relates to 3-O-glycosyl 16-membered macrolide antibacterials, such as 2″,4″,4‴-tri-O-acetyl-O-(4′-O-phenoxyacetyl-α-L-cladinosyl)-(1→3)-desmycosin and O-α-L-cladinosyl-(1→3)-12,13-dehydro-12,13-deoxorosaramicin, related derivatives, pharmaceutically acceptable acid addition salts thereof, pharmaceutical compositions containing them and a method of eliciting an antibacterial response using the novel antibacterials or pharmaceutical compositions containing them.

Several 3-O-glycosyl derivatives are found among the naturally occurring 12-and 14-membered macrolides. For example, among the 12-membered macrolides, methymycin is disclosed in J. Am. Chem. Soc., 1956, 78, p. 6390, and neomethymycin is disclosed in Tetrahedron, 1958, 3, p. 255. Among the 14-membered macrolides, erythromycin is disclosed in J. Am. Chem. Soc., 1957, 79, p. 6062; oleandomycin is disclosed in J. Am. Chem. Soc., 1960, 82, p. 3225; and lankamycin is disclosed in J. Am. Chem. Soc., 1970, 92, P. 4129. However, there is no disclosure of 3-O-glycosyl 16-membered macrolide antibacterials available synthetically or occuring naturally.

This invention may be summarized as a compound represented by formula I:

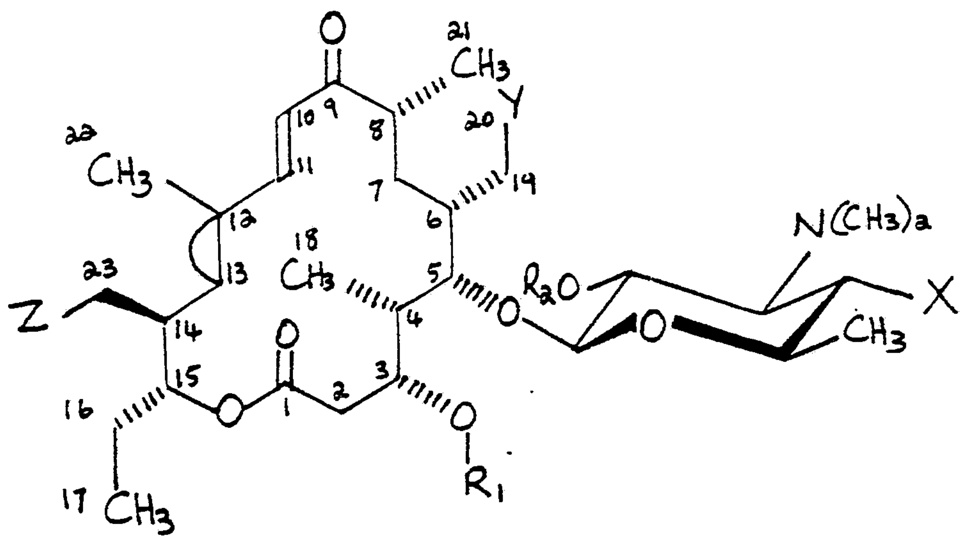

I

wherein $R_1$ is a glycosyl group, represented by the formula II:

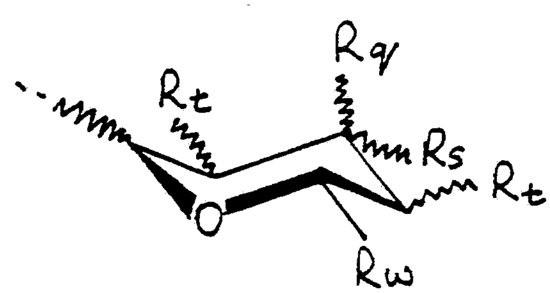

II

wherein $R_q$ and each $R_t$ are independently hydrogen, hydroxy, acyloxy, loweralkoxy, aralkoxy, N-loweralkylamino-carbonyloxy, N-aralkylamino carbonyloxy, or arylsulfonyloxy ($-OSO_2Ar$) and $R_s$ and $R_w$ are independently hydrogen, lower alkyl or $-CH_2OH$
or represented by the formula IIA:

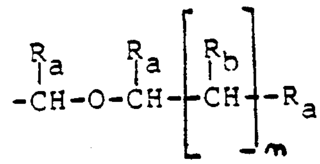

IIA

wherein each $R_a$ is independently hydrogen, lower alkyl, or $CH_2OH$ and each $R_b$ is independently hydrogen, hydroxy, acyloxy, lower alkoxy, aralkoxy, N-loweralkylaminocarbonyloxy, N-aralkylaminocarbonyloxy or arylsulfonyloxy and n is 1, 2, 3, or 4;

$R_2$ is hydrogen or an acyl group;

X is hydrogen, hydroxy, an acyloxy group

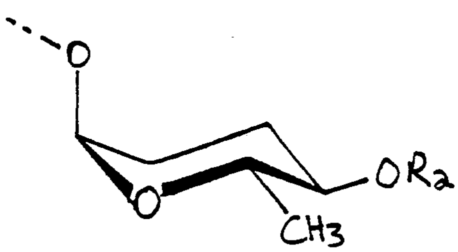

or

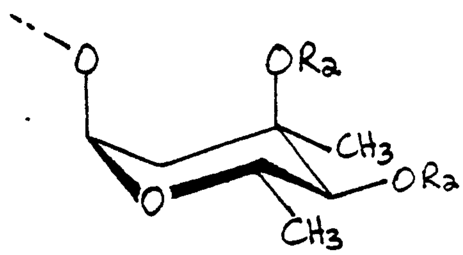

wherein each $R_2$ is independently as defined hereinabove;

Y is hydrogen, a formyl group, methyl,

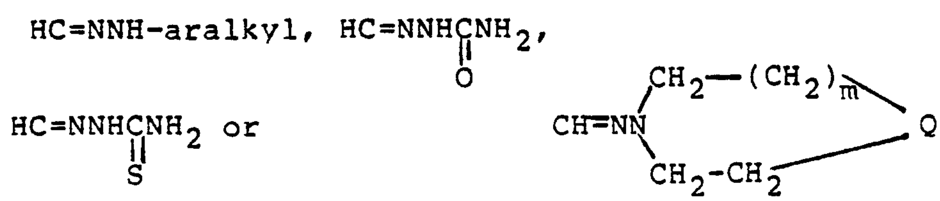

wherein m is 0,1, or 2 and Q is independently $CR_3R_4$, $NR_3$, O, S, $SO_2$, $CR_3OR_4$,

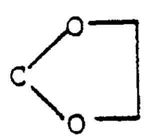

,

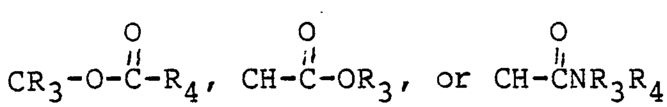

wherein $R_3$ and $R_4$ are independently hydrogen, lower alkyl, aralkyl, G-substituted aralkyl, aryl and G-substituted aryl wherein G is independently one or more of halogen, trifluoromethyl, lower alkoxy or ($C_2$-$C_7$) alkanoyl;

Δ is a 12,13 double bond or a 12,13-oxo moiety; Z is hydrogen, hydroxy, an acyloxy group, a N,N-di(loweralkyl)amino group,

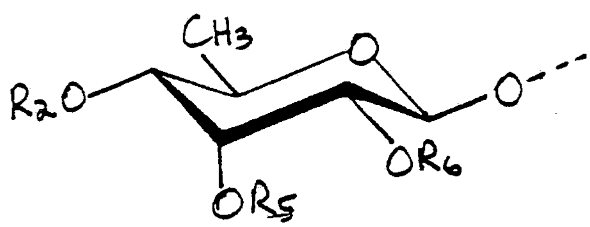

,

loweralkoxycarbonyloxy, aralkoxycarbonyloxy, N-loweralkylaminocarbonyloxy, or N-aralkylaminocarbonyloxy wherein $R_2$ is defined above and $R_5$ and $R_6$ are independently hydrogen, lower alkyl or acyl groups; or a pharmaceutically acceptable acid addition salt thereof.

In a preferred class of compounds, $R_1$ is an α-L-glycosyl group represented by formula II.

In an other preferred class of compounds $R_1$ is α-L-clandinosyl or tetrahydropyranyl,

X is hydrogen, hydroxy, or

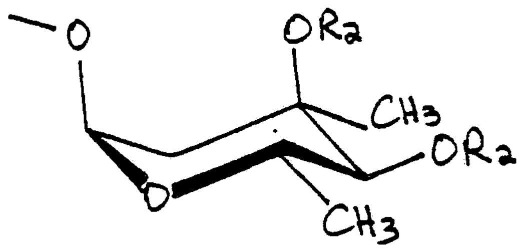

wherein $R_2$ is as defined previously,

Y is menthyl or formyl,

Z is hydrogen, acyloxy, hydroxy, or

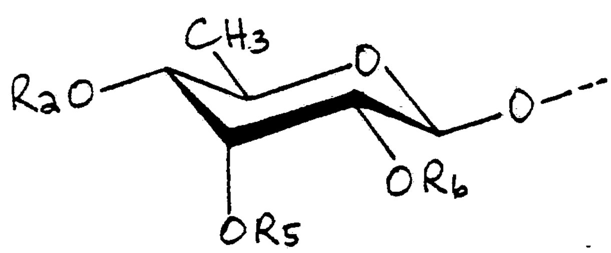

wherein $R_2$, $R_5$ and $R_6$ are as defined previously.

In still another preferred class of compounds, $R_1$ is

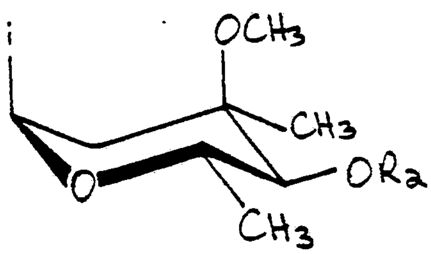

wherein $R_2$ is as defined previously,

X is hydroxy or acyloxy,

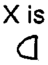 is a 12,13 double bond, and

Z is

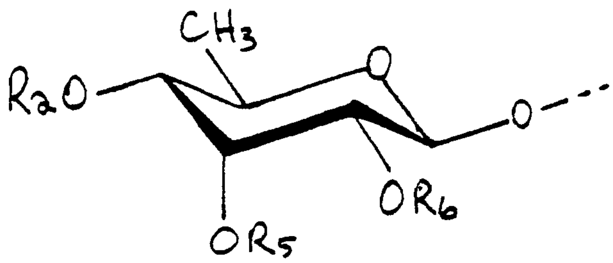

wherein $R_2$, $R_5$ and $R_6$ are as defined previously. Especially compounds within the class are those wherein $R_1$ is

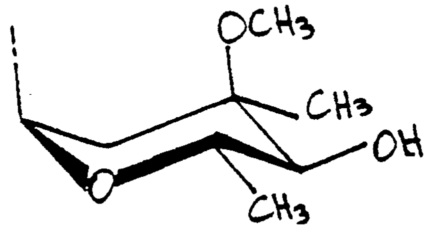

or

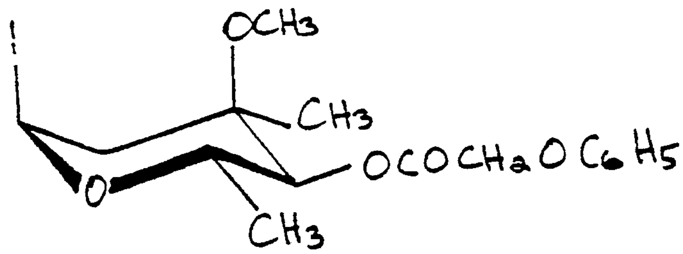

X is hydroxy or acetyl,
Z is

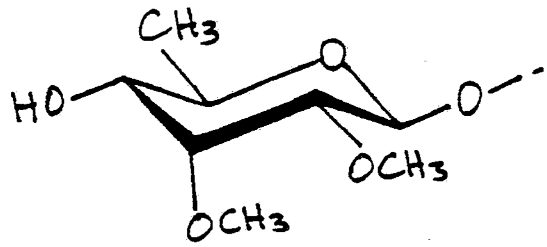

or

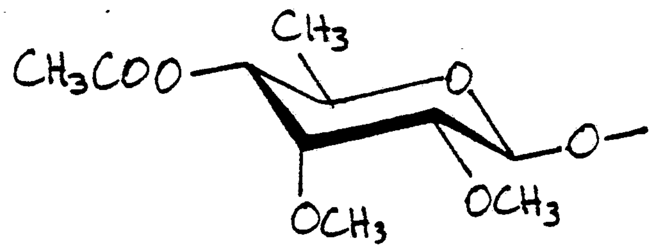

and

R is acetyl.

Preferred individual compounds are

O-(α-L-cladinoxyl)-(1→3)-12,13-dehydro-20-dihydro-12,13,20-dideoxorosaramicin,
O-(α-L-clandinosyl)-(1→3)-12,13-dihydro-12,13-deoxorosaramicin,
O-(4′-O-acetyl-α-L-cladinosyl)-(1→3)-2″-O-acetyl-12,13-dehydro-20-dihydro-12,13,20-dideoxorosaramicin,
O-(4′-O-acetyl-α-L-cladinosyl)-(1→3)-2″-O-acetyl-12,13-dehydro-12,13-deoxorosaramicin,
O-tetrahydropyranyl-(1→3)-rosaramicin,
2″,4″,4‴, tri-O-acetyl-19-deformyl-O-(4′-O-phenoxyacetyl-α-L-cladinosyl)-(1→3)-demycosin,
2″,4″,4‴-tri-O-acetyl-O-(4′-O-phenoxyacetyl-α-L-cladinosyl)-(1→3)-desmycosin,
2″,4″,4‴-tri-O-acetyl-20-deoxy-20-dihydro-O-(4′-O-phenoxyacetyl-α-L-cladinosyl)-(1→3)-desmycosin,
O-(α-L-cladinosyl)-(1→3)-19-deformyldesmycosin,
O-(α-L-cladinosyl)-(1→3)-20-deoxo-20-dihydrodesmycosin,
O-α-L-cladinosyl-(1→3)-demycosin.

The invention also includes the use of a compound of formula I for the preparation of a pharmaceutical composition useful for treating bacterial infection and a method for making a pharmaceutical composition comprising mixing a compound of formula I with a pharmaceutically acceptable carrier.

The compounds of this invention show high serum levels and are useful as antibacterials against susceptible bacterial infections, especially those caused by Gram-positive organisms.

This invention also provides a pharmaceutical composition comprising an antibacterially effective amount of a compound represented by formula I in admixture with a pharmaceutically acceptable carrier therefor.

This invention further provides a method of eliciting an antibacterial effect against susceptible bacterial infections, wherein an antibacterially effective amount of a compound represented by formula I or pharmaceutical compositions thereof is administered to an animal having a susceptible bacterial infection.

The compounds of this invention have stereochemical configuration indicated for the structure of compounds of formula I, and it is to be understood that the stereochemical configuration is identical to that of tylosin. The stereochemistry of the 12,13-oxo moiety, when present in the compounds of formula I, is indicated below:

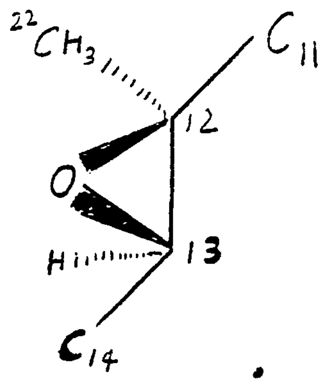

The term "acyl group" as used herein means ($C_2$-$C_7$)alkanoyl; ($C_2$-$C_7$)alkanoyl substituted by chloro, lower alkoxy, aryl, aralkoxy, or aryloxy; aroyl; and substituted aroyl; said aroyl substituents being one or more of halogen, nitro, lower alkoxy, or lower alkyl.

The term "acyloxy" means the "acyl group" as defined herein univalently bonded to divalent oxygen and includes inter alia, acetyloxy, butyryloxy, iso-valeryloxy, phenylacetyloxy, benzoyloxy, p-methoxybenzoyloxy.

The term "lower alkyl" as used herein means straight and branched-chain alkyl groups of one to six carbons including methyl, ethyl, propyl, butyl, pentyl, hexyl and the corresponding branched-chain isomers thereof such as iso-propyl, tert-or sec-butyl, iso-valeryl and iso-hexyl. Methyl is the preferred alkyl group.

The term "lower alkoxy" means "lower alkyl" groups univalently bonded to divalent oxide and includes inter alia, methoxy, ethoxy, and propoxy.

The term "aryl" means phenyl and biphenyl optionally substituted by lower alkyl.

The term "halogen" means fluoro, chloro and bromo, preferably fluoro and chloro.

The term "aralkyl" means lower alkyl substituted by aryl including benzyl, α-and β-phenethyl and α-and β-(o-tolyl)ethyl.

"Aralkoxy" refers to "aralkyl" as defined hereinabove univalently bonded to divalent oxygen.

The term "aryloxy" refers to phenoxy and biphenyloxy.

The term "($C_2$-$C_7$)alkanoyl" means carbonyl groups univalently bonded to "lower alkyl" groups and includes acetyl, propionyl, butyryl, iso-butyryl and iso-valeryl. The preferred ($C_2$-$C_7$) alkanoyl groups are acetyl, butyryl, iso-valeryl for $R_1$, $R_2$, $R_3$, $R_4$, $R_5$, and $R_6$.

Typical suitable chloro-substituted ($C_2$-$C_7$) alkanoyl groups include 2-chloro($C_2$-$C_7$)alkanoyl such as 2-chloroacetyl and 2-chloropropanoyl. Typical suitable lower alkoxy ($C_2$-$C_7$) alkanoyls include methoxyacetyl, ethoxyacetyl, 2-and 3-methoxypropanoyl, and propoxyacetyl. Typical suitable aryl-substituted ($C_2$-$C_7$) alkanoyl groups include phenylacetyl and biphenylacetyl. Typical suitable aryloxy-substituted ($C_2$-$C_7$)-alkanoyls include phenoxyacetyl and biphenyloxyacetyl.

The term "aroyl" means substituted and unsubstituted benzoyl wherein the substituents are one or more of halogen, nitro, lower alkoxy or lower alkyl. Typical suitable substituted benzoyl include p-fluoro benzoyl, 3-chlorobenzoyl, 4-nitrobenzoyl, 3-nitrobenzoyl, p-ethoxybenzoyl, 3-chloro-4-nitrobenzoyl, 3-fluoro-4-methoxybenzoyl, 3-nitro-4-ethylbenzoyl and 3-chloro-4-methylbenzoyl.

Typical suitable G-substituted aryl groups include 4-fluorophenyl, 3-chlorophenyl, 4-trifluoromethylphenyl, 4-ethoxyphenyl, 4-acetylphenyl and 3-chloro-4-methoxyphenyl.

Typical suitable G-substituted aralkyl groups include 4-fluorobenzyl, 3-chlorophenylethyl, 4-methoxy-α-phenethyl, and 4-trifluoromethylbenzyl.

The term "N,N-di(loweralkyl)amino" means two "lower alkyl groups", each univalently bonded to a trivalent nitrogen atom, including dimethylamino, diethylamino, di-n-propylamino, methylpropylamino and diisopropylamino.

The term "lower alkoxycarbonyloxy" as used herein means "lower alkoxy" groups univalently bonded to the carbonyl carbon of the carbonyloxy moiety

$$(-\overset{O}{\overset{\|}{C}}-O-),$$

including inter alia, methoxycarbonyloxy, ethoxycarbonyloxy and n-and iso-propoxycarbonyloxy.

The term "aralkoxycarbonyloxy" as used herein means an "aralkyl group" univalently bonded to a divalent oxide which in turn is univalently bonded to the carbonyl carbon of the carbonyloxy moiety including phenethoxycarbonyloxy, benzyloxycarbonyloxy, and o-tolylethoxycarbonyloxy.

The term "N-loweralkylaminocarbonyloxy" as used herein means one or two "lower alkyl group" univalently bonded to the trivalent amino moiety, -N-which in turn is univalently bonded to the carbonyl carbon of a carbonyloxy moiety, including N-methylaminocarbonyloxy N,N-dimethylaminocarbonyloxy, N-iso-propyl aminocarbonyloxy N,N-methylethylaminocarbonyloxy and N-tert-butylaminocarbonyloxy.

The term "N-aralkylaminocarbonyloxy" as used herein means one or two "aralkyl group" univalently bonded to a trivalent nitrogen, i.e. -N-which in turn is univalently bonded to the carbonyl carbon of the carbonyloxy moiety, including N-benzylaminocarbonyloxy, N-(β-phenethyl)aminocarbonyloxy and N-o-tolylethylaminocarbonyloxy.

The term "arylsulfonyloxy" means "aryl" univalently bonded to the sulfur in sulfonyloxy including phenylsulfonyloxy and biphenyl sulfonyloxy.

In formula IIA, $R_a$ is preferably hydrogen or methyl. $R_b$ is preferably hydrogen or loweralkoxy.

Typical suitable acyclic glycosyl groups represented by formula IIA include $-CH_2-O-CH_2CH_3$, $-CH(CH_3)-O-CH(CH_3)_2$, $-CH_2-O-(CH_2)_2CH_3$, $-CH_2-O-(CH_2)_3-CH_3$, $-CH(CH_3)-O-CH_2CH(CH_3)_2$, $-CH_2-O-(CH_2)_4-CH_3$, $-CH-(CH_3)-O-CH(CH_3)-(CH_2)_3-CH_3$, $-CH_2-O-CH_2-CH(OCH_3)CH_3$, $-CH_2-O-CH(CH_2OH)-CH_3$ or $-CH(CH_3)-O-CH-(CH_2OH)(CH_2)_3CH_3$.

$R_q$ which may be in the axial (a) position (formula IIB) or equatorial (e) position (formula IID) and is preferably hydroxy, methoxy or acyloxy. $R_t$ is preferably hydrogen, hydroxy, lower alkoxy, for example methoxy, or acyloxy, for example acetyloxy, butyryloxy or iso-valeryloxy. $R_s$ and $R_w$ may be in the axial or equatorial position. $R_s$ is preferably hydrogen or methyl; $R_w$ is preferably methyl.

The preferred glycosyl groups include the following α-L-glycosyl groups represented by formulas IIB, IIC and IID

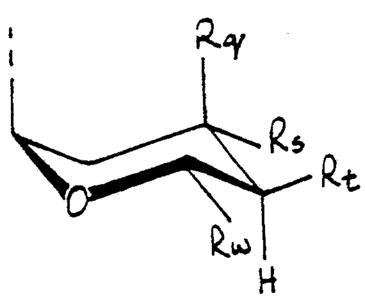

IIB

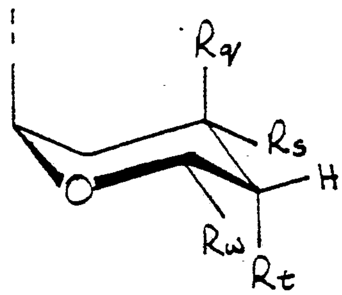

IIC

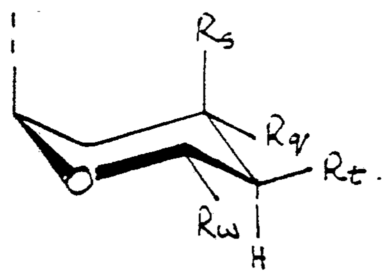

IID

wherein $R_s$ and $R_w$ are independently hydrogen or methyl and wherein $R_q$ is hydroxy, lower alkoxy or lower acyloxy and $R_t$ is hydrogen, hydroxy, lower alkoxy or acyloxy:

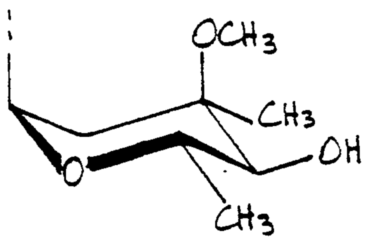
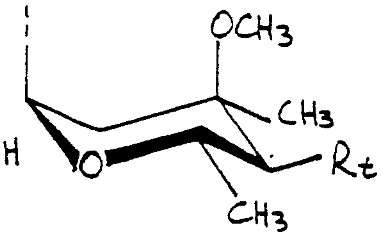
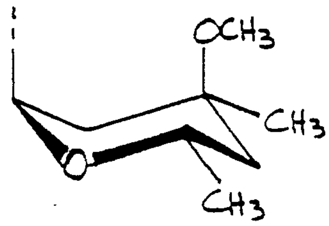

α-L-cladinosyl

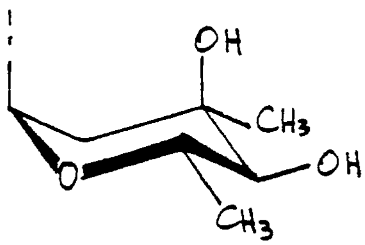
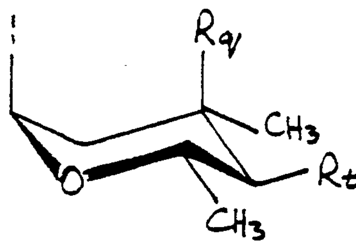
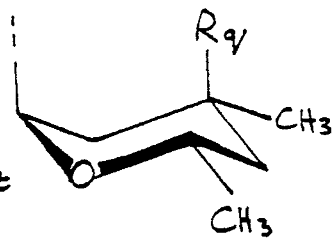

α-L-mycarosyl

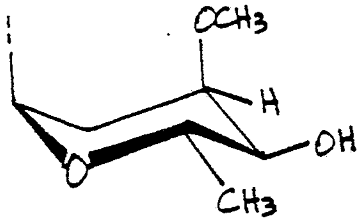 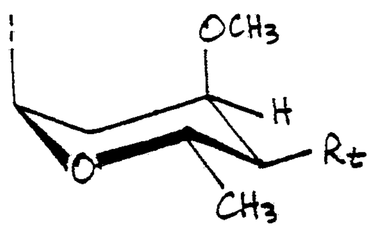 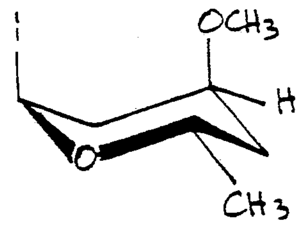

3-O-methyl-α-L-digitoxosyl

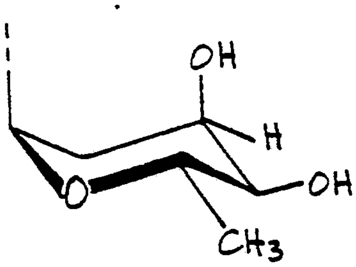 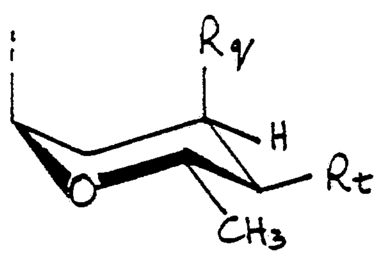 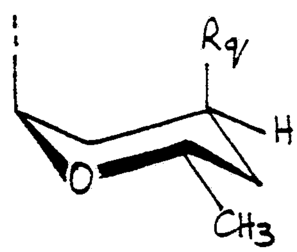

α-L-digitoxosyl

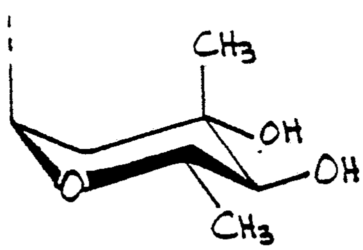 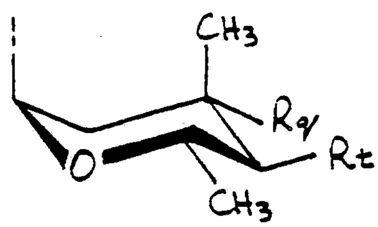 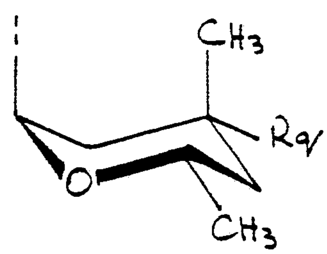

α-L-3-epi-mycarosyl

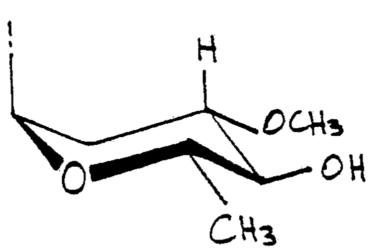 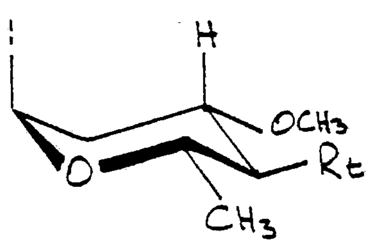 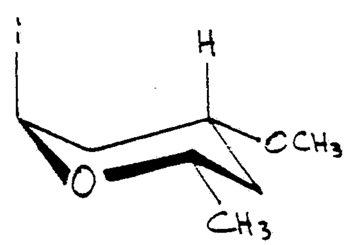

α-L-oleandrosyl

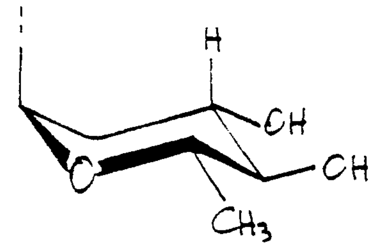

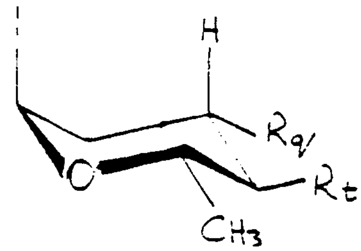

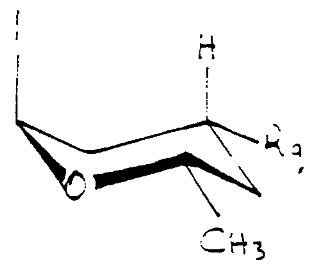

α-L-olivosyl

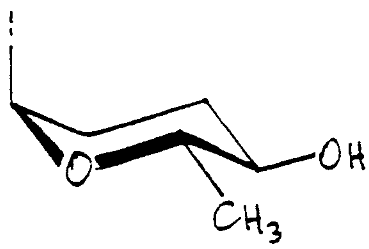

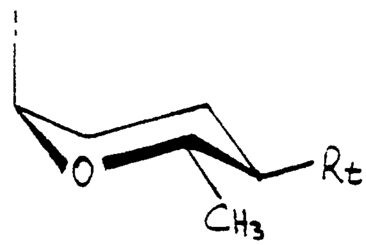

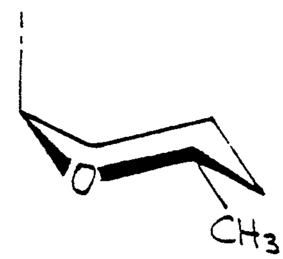

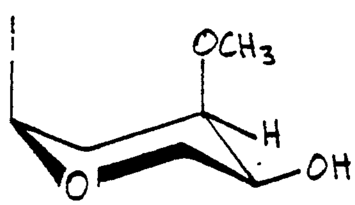

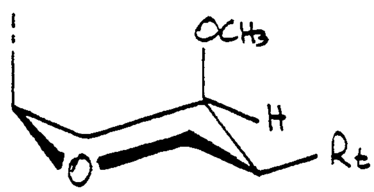

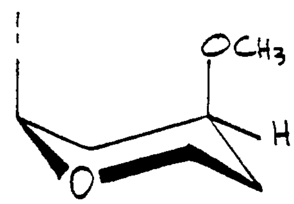

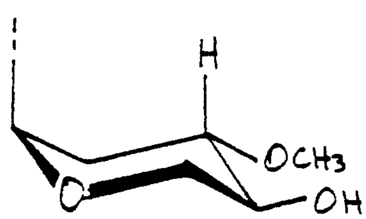

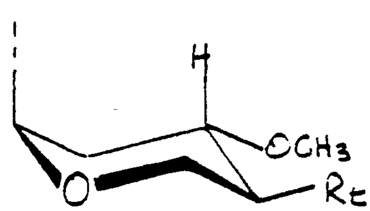

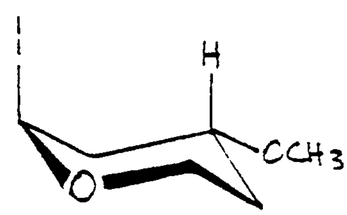

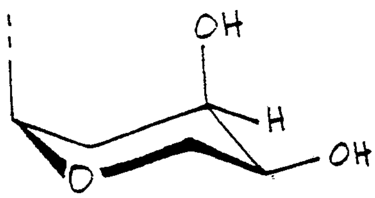
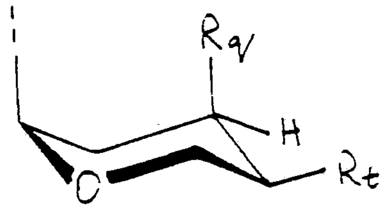
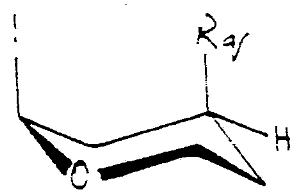

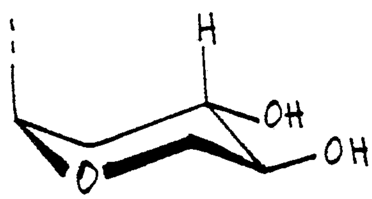
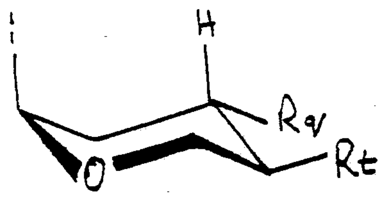
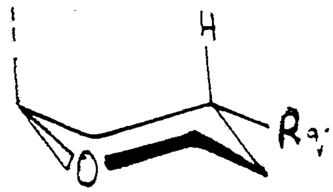

as well as 4-epi derivatives of all of the above gylcosyl groups.

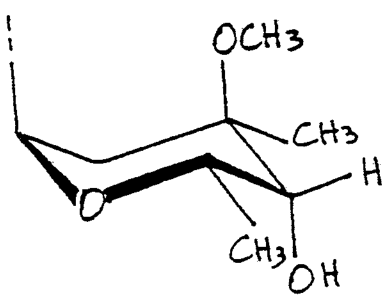
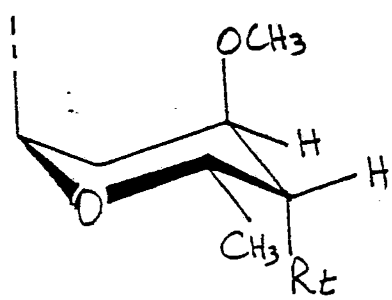

α-L-arcanosyl

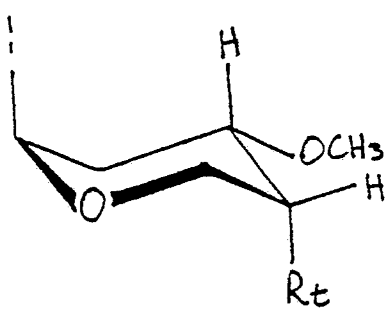

The corresponding β-L-glycoside groups and α-or β-3-tetrahydropyranyl group, i.e.,

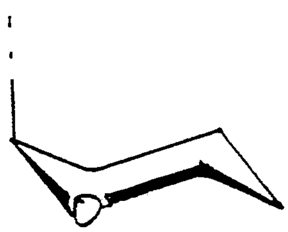
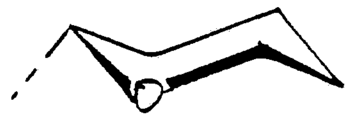

may also be used.

The more preferred α-L-glycosyl groups of formula IIB, IIC, and IID are α-L-cladinosyl, 4-O-acyl-α-L-cladinosyl, α-L-digitoxsyl, 3-O-methyl-α-L-digitoxsyl, 3,4-di-O-acyl-α-L-digitoxsyl, 3,4-di-O-acyl-α-L-mycarosyl,4-O-acyl-α-L-oleandrosyl, and 3-tetrahydropyranyl.

The α-L-cladinosyl group may be obtained by acid cleavage of erythromycin-A [See Example 1(a)]. The preparations of α-L-digitoxose and 3-O-methyl-α-L-digitoxose are disclosed by J.S. Brimacombe et al. in J. Chem. Soc., Perkin Trans I, 1982, pp 2583-2587; and the preparations of α-L-and α-L-3-epi-mycarose, α-L-oleandrose, α-L-olivose, α-L-cladinose, α-L-digitoxose and α-L-arcanose as well as the pentoses are disclosed in The Carbohydrates, Chemistry and Biochemistry, 1980, 2nd Edition (W. Pigman et al., Editors), Chapter 17 (N.R. Williams et al.), pp 761-798 Academic Press, New York.

Typical suitable starting materials for preparation of the compounds of this invention include any of the known 16-membered macrolides having a free or potentially free hydroxy group at the C-3 carbon as defined in formula I and which may possess other reactive tertiary, secondary or primary hydroxy groups suitably protected as described hereinafter.

Typical suitable starting materials useful in preparation of the compounds of the present invention include the following known antibiotics or those that are readily preparable from known antibiotics. The preparation of desmycosin and that of tylosin are described by R. L. Hamill et al. in U.S. Patent No. 3,178,341. The preparation of macrocin and that of lactenocin are described by R. L. Hamill et al. in U.S. Patent No. 3,326,759. Cirramycin $A_1$ is prepared as described by H. Koshiyama et al., J. Antibiotics Ser. A16, 59-66 (1963) and S. Nash et al. in U.S. Patent No. 4,252,898. Antibiotic M-4365 $G_2$ (repromicin) can be prepared by the method described by T. Furumai et al. in J. Antibiotics, 30, 443-449 (1977) or as described by A. K. Ganguly in U.S. Patent No. 3,975,372. The preparation of 9-dihydrodesmycosin (dihydrodesmycosin) is described by E.H. Massey in U.S. Patent No. 3,769,273. 9-Dihydrolactenocin is prepared in an analogous manner. Rosaramicin can be prepared as described by M. J. Weinstein et al. in U.S. Patent No. 4,234, 690. The preparation of 12,13-deoxo-12,13-dehydrorosaramicin (M-4365$A_2$) and that of 20-deoxo-20-dihydro-12,13-deoxo-12,13-dehydrorosaramicin are described in Examples 1 and 28, respectively, of U.S. Patent No. 4,056,616. The preparation of 23-demycinosyltylosin (DMT), 20-dihydro-DMT, 5-O-mycaminosyltylonolide (OMT) and of 20-dihydro-OMT are described in U.S. Patent No. 4,321,361. The preparation of 23-deoxy-5-O-mycaminosyltylonolide (DOMT), 23-de(mycinosyloxy)tylosin (DMOT), 20-dihydrodesmycosin, and of 20-dihydrolactenocin are described in U.S. Patent No. 4,357,325.

The preparation of 20-deoxo-20-dihydroDMOT is described in U.S. Patent No. 4,528,369. The production of 16-membered macrolide antibiotics CP-56,064 and CP 56,063 by fermentation of Streotomyces albus is described in U.S. Patent No. 4,454,314.

Other typical suitable starting materials may be prepared from the antibiotics listed hereinabove by use of known synthetic techniques. Thus, for example, the formyl group in desmycosin, DMOT, tylosin, 12,13-deoxo-12,13-dehydrorosaramicin or DMT can be replaced by hydrogen by use of tris(triphenylphosphine) rhodium chloride, in accordance with the procedure described in U.S. Patent No. 4,345,069, to give the corresponding 19-deformyl derivative, e.g. 19-deformyldesmycosin. In addition, epoxidation of the 12,13 double bond in for example, tylosin, DMT, 19-deformyl DMT, DMOT or 19-deformyl DMOT may be performed using m-chloroperbenzoic acid as described in U.S. Patent No. 4,477,443 (Examples 58-61) to give the corresponding 12,13-oxo derivative, e.g. 12,13-oxotylosin, 12,13-oxo DMT, 19-deformyl-12,13-oxoDMT, 12,13-oxo DMOT and 19-deformyl-12,13-oxoDMOT. The production of 12,13-oxo DMOT (antibiotic M119a) by fermentation of alkalophilic Actinomycetes is disclosed by H. Tanaba et al. Paper #1150 25th ICAAC, Minneapolis, MN 29 Sept. to 2 Oct. 1985.

Additional suitable starting materials useful for preparation of the compounds of this invention may be obtained by modification of the suitable starting materials hereinabove such as 19-deformyl DMT by well known techniques to convert the free hydroxy group at the 23-carbon in such compounds into compounds wherein the 23-carbon is attached to acyloxy-N,N-di(lower alkyl)amino, lower alkoxycarbonyloxy, aralkoxycarbonyloxy, N-loweralkylaminocarbonyloxy or N-aralkylaminocarbonyloxy.

Compounds of the present invention are made by reacting a compound having the formula

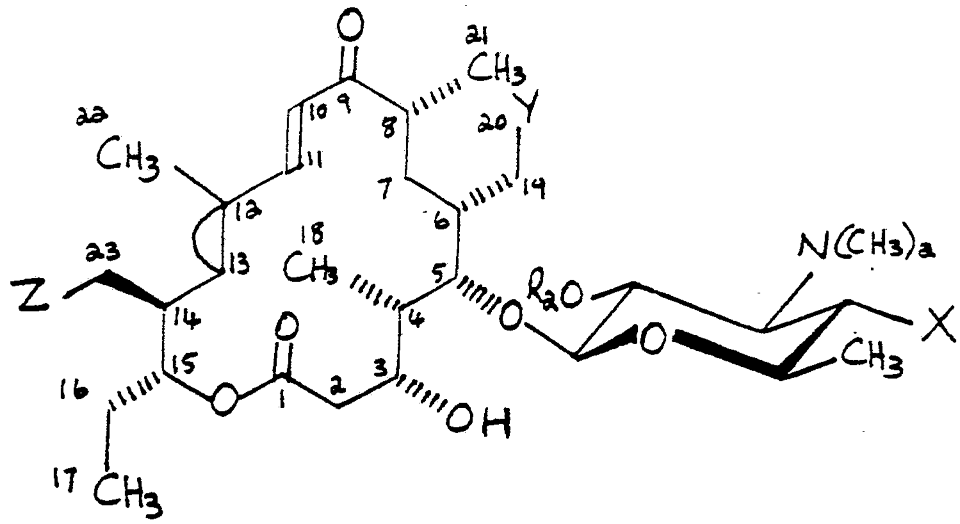

wherein X, Y, Z and $R^2$ are as previously defined with a compound of the formula

L - $R_1$

wherein L is a leaving group and $R_1$ is as previously defined,

wherein any reactive groups are protected, if necessary or desired, followed if necessary or desired by one or more of the following steps:

(i) removal of any protecting group,

(ii) conversion of the compound so formed to a different compound of formula I, and/or

(iii) formation of a pharmaceutically acceptable acid addition salt of the compound of formula I.

The preferred leaving group, L, is pyridyl-2-thio.

The Glycosylation Reaction

The glycosyl groups are attached to the free hydroxyl group at the C-3 carbon of the 16-membered macrolide starting materials by use of the procedures known in the art. For example, the procedure of Tatsuta, Carb. Research 1977, 54, p. 85 or of R.B. Woodward et al., J. Am. Chem. Soc. 1981, 103, p. 3215 may be used.

Generally, a suitably protected glycosyl group, e.g., 1-deoxy-1-(pyridyl-2-thio)-α and/or β-L-glycoside such as the 1-S-pyridyl derivative of L-cladinose is reacted with a 16-membered macrolide having a free hydroxyl group at the C-3 carbon (and with other reactive tertiary, secondary and/or primary hydroxyl groups suitably protected) in the presence of anhydrous silver perchlorate or anhydrous silver trifluoromethylsulfonate in an anhydrous aprotic solvent such as acetonitrile. Reaction temperatures of 20-25°C for 20-30 hours are conveniently used.

Prior to the glycosylation of the free hydroxy group at the C-3 carbon, any other free hydroxy groups are conveniently protected as acyl derivatives by use of ($C_1$-$C_5$)alkanoic anhydrides, such as, acetic anhydride in the presence of added base such as pyridine, or as the silyl ethers. Standard deprotection techniques which are well known are used to remove the acyl or silyl protecting groups. When the macrolide has a lactone moiety which is sensitive to base-catalyzed ring opening during the deprotecting procedures used to remove, for example, the acyl protecting groups, more labile acyl protecting groups such as aryloxyacyl, haloacetyl, alkoxyacetyl, formyl or haloalkoxycarbonyl may be used. Since very mild basic conditions are employed to remove these more labile acyl groups, the lactone moiety is not adversely affected. In this manner, more labile protecting groups such as phenoxyacetyl may be used for protecting the hydroxyl groups in the glycoside group. For example, phenoxyacetyl chloride in the presence of pyridine is reacted with L-digitoxose to form 1,3,4-tri-O-phenoxyacetyl-α-and -β-L-digitoxose. The 1-O-phenoxyacetyl group is removed by treatment at ambient temperatures, e.g., 25°C with aqueous HCl e.g. 0.1N HCl The product so formed is reacted with 2,2-dipyridyldisulfide in the presence of tri-n-butylphosphine to give 3,4-di-O-phenoxyacetyl-1-deoxy-1-(pyridyl-2-thio)-α and/or β-L-digitoxose.

Exemplary of the preparation of compounds of the present invention is the following process for the preparation of 3-O-α-and/or-β-L-cladinosyl-12,13-deoxo-12,13-dehydrorosaramicin and the corresponding 19-deformyl and 20-deoxo-20-dihydro compounds of formula Ia wherein Y = CHO, H and $CH_3$, respectively.

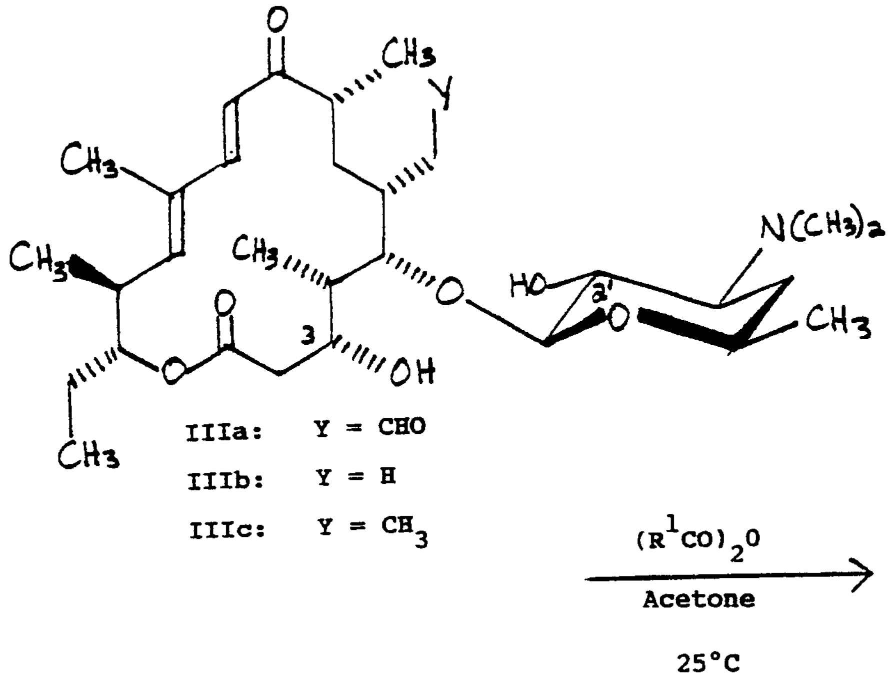


IIIa: Y = CHO

IIIb: Y = H

IIIc: Y = $CH_3$

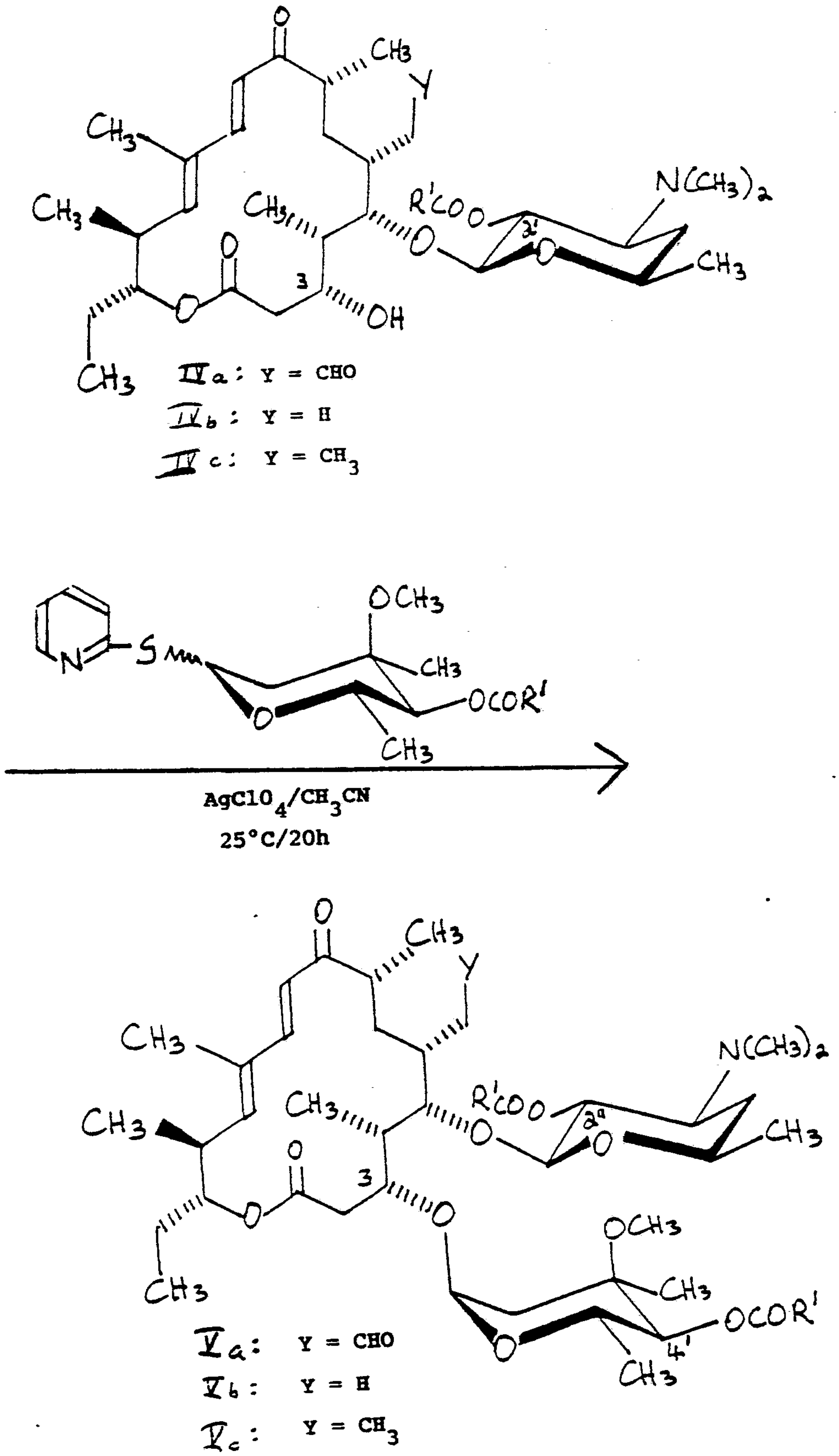
IVa : Y = CHO
IVb : Y = H
IVc : Y = $CH_3$
$AgClO_4/CH_3CN$
25°C/20h
Va : Y = CHO
Vb : Y = H
Vc : Y = $CH_3$

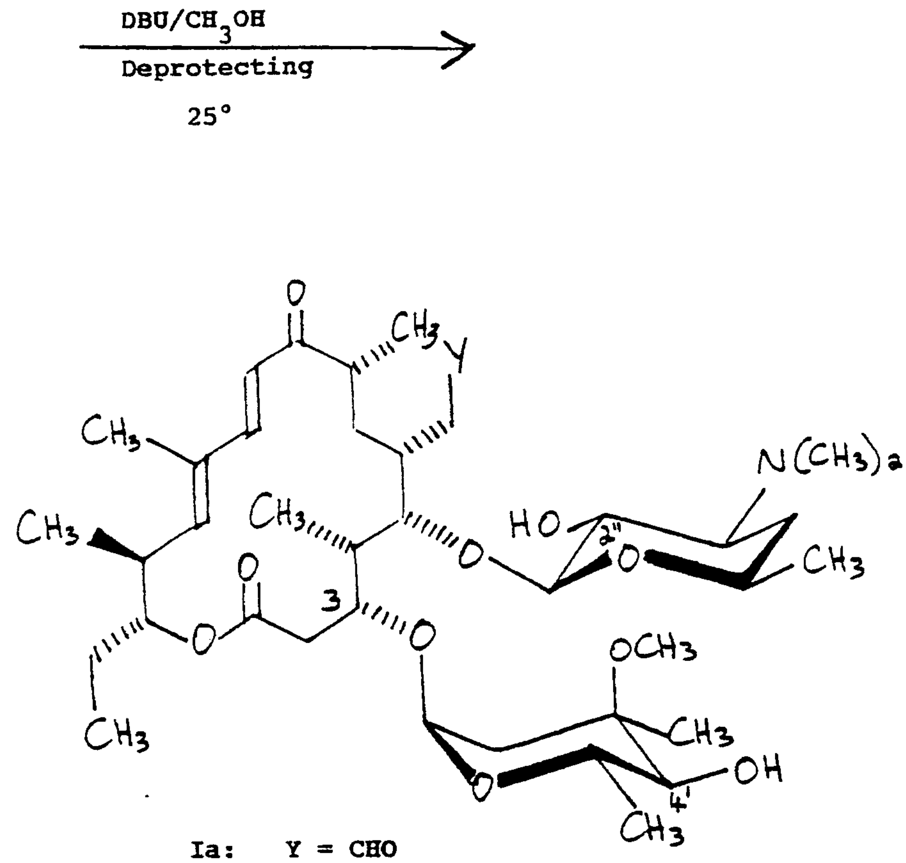


Ia: Y = CHO

Ib: Y = H

Ic: Y = $CH_3$

As illustrated in the above schematic, .12,13-deoxo-12,13-dehydrorosaramicin or the corresponding 19-deformyl-and 20-deoxo-20-dihydro derivatives represented by formulas IIIa-c wherein Y = CHO, H and $CH_3$, respectively, is acylated at the hydroxy on the 2′ carbon by use of a lower alkanoic acid anhydride, (R′CO)-$_2$O, normally acetic anhydride in acetone at 25°C to form the 2′-O-acyl 12,13-deoxo-12,13-dehydrorosaramicin represented by formula IVa. Compound IVa is reacted with 4-O-acyl-1-deoxy-1-(pyridyl-2-thio)-α-and/or -β-L-cladinoside in the presence of anhydrous silver perchlorate or anhydrous silver trifluoromethylsulfonate in anhydrous acetonitrile at 25°C for 20 hours to provide the O-(4′-O-acyl-α-L-cladinosyl)-(1→3)-12,13-dehydro-12,13-deoxorosaramicin, compound Va (or the corresponding deformyl or 20-deoxo-20-dihydro rosaramicin derivatives). The acyl groups in compound Va-c are removed from the 2″ hydroxyl group in the desosaminyl sugar and the 4′-hydroxyl group in the cladinosyl sugar by treating compound Va-c with an organic trisubstituted nitrogen base for example, 1,8-diazabicyclo[5.4.0]undec-7-ene (DBU) and a lower alkanol, e.g., methanol to give compounds of formula Ia:Y = CHO, Ib: Y = H or Ic:Y = $CH_3$ and X = Z = H.

As used herein, the term "organic trisubstituted nitrogen base" refers to acyclic tertiary amines, pyridine and lower alkyl substituted pyridines and bicyclic tertiary amines. Typical suitable acyclic tertiary aliphatic amines include tri(lower alkyl) amines such as trimethylamine, triethylamine, tri-n-propylamine and dimethyl-sec-butylamine. Triethylamine is the preferred acyclic tertiary amine. Typical suitable substituted pyridines include 2-, 3-and 4-methylpyridine. Typical suitable bicyclic tertiary amines include 1,5-diazabicyclo[4.4.0]non-5-ene, hereinafter "DBN" and 1,8-diazabicyclo[5.4.0]undec-7-ene, hereinafter "DBU".

The term "lower alkanol" refers to straight and branched-chain alcohols of one to six carbons such as methanol, ethanol, n-and iso-propanol. Methanol is preferred.

The 4-O-acylcladinosyl derivatives (VIa-c) of the compounds of formulas Ia-c are prepared by selectively removing the acyl group from the 2″ position of the desosaminyl sugar by treating compounds Va-c with a lower alkanol, normally methanol, at 25°C for 16 hours.

V a-c

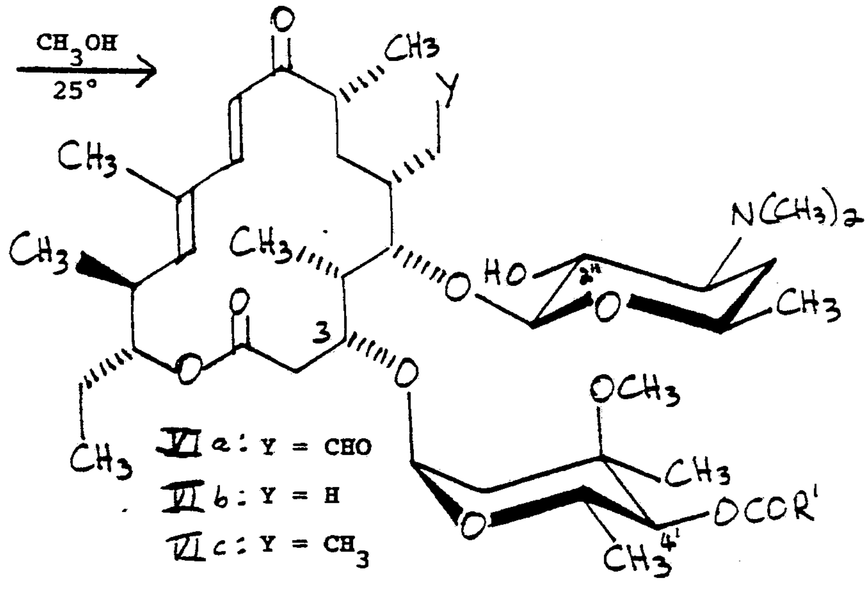


VIa: Y = CHO
VIb: Y = H
VIc: Y = $CH_3$

Using procedures similar to those outlined for the preparation of 3-O-α-glycosyl 16-membered macrolide antibacterials of this invention, the 3-O-β-glycosyl 16-membered macrolide antibacterials of this invention wherein the glycosyl group, $R_1$, is represented by formula II may be prepared as described in Example 20.

The 3-O-glycosyl 16-membered macrolide antibacterials of this invention wherein the glycosyl group, $R_1$, is represented by the formula IIA may be prepared as described in Example 18.

This invention also relates to a pharmaceutical composition comprising a compound of the present invention or its pharmaceutically acceptable acid addition salts in admixture with a pharmaceutically acceptable carrier.

The compounds of the present invention are capable of forming pharmaceutically acceptable acid addition salts with inorganic and organic acids. The term "pharmaceutically acceptable acid addition salts" means those salts that do not exhibit toxic manifestations at normal therapeutic doses. Exemplary of such salts are those formed by reaction of the compounds of this invention with acids such as hydrochloric, sulfuric, phosphoric, citric, acetic, propionic, tartaric, maleic, benzoic, cyclopropylcarboxylic, adamantylcarboxylic, lauryl sulfonic, glucoheptonic, stearic, lactobionic and the like. Pharmaceutically acceptable acid addition salts may be prepared by methods generally used in the art such as by adding a stoichiometric amount of acid to a solution of a compound of the invention in an inert organic solvent and isolating the salt by art known methods such as precipitation of the salt with a solvent wherein the salt is not appreciably soluble, e.g. diethyl ether. An inert organic solvent is one which does not react with the antibacterial, the acid or the salt under conditions of the reaction.

Typical pharmaceutically acceptable carriers suitable for use in the formulations described are exemplified by sugars such as lactose, sucrose, mannitol and sorbitol; starches such as corn starch, tapioca starch and potato starch; cellulose and derivatives such as sodium carboxymethyl cellulose, ethyl cellulose and methyl cellulose; calcium phosphates such as dicalcium phosphate and tricalcium phosphate; sodium sulfate; calcium sulfate; polyvinyl pyrrolidone; polyvinyl alcohol; stearic acid; alkaline earth metal stearates such as magnesium stearate; vegetable oils such as peanut oil, cottonseed oil, sesame oil, olive oil and corn oil; non-ionic, cationic and anionic surfactants, ethylene glycol polymers; betacyclodextrin; fatty acids, hydrolyzed cereal solids; water, polyalkylene glycols; gums; and petroleum ; as well as other non-toxic compatible fillers, binders, and lubricants commonly used in pharmaceutical formulations. The compositions may also contain preservatives, areosol propellants and coloring, thickening, suspending, dispensing, emulsifying, wetting, stabilizing and buffering agents.

This invention also embraces a method of eliciting an antibacterial effect against susceptible bacterial infections which comprises administering to an animal, preferably a warm-blooded animal such as man having a susceptible bacterial infection an antibacterially effective amount of a compound of formula I or a pharmaceutical composition thereof.

In order to elicit an antibacterial effect against a susceptible bacterial infection, the compounds of this invention may be administered orally, intramuscularly, topically or intraveneously. Administration may be effected by any of the conventional methods, i.e., by the use of tablets, capsules, and suspensions,

solutions, creams, ointments or injectables. Each of the dosage forms can be formulated utilizing non-toxic pharmaceutically acceptable excipients conventionally known in the art. The compounds of this invention are preferably administered intravenously at doses of from about 5 to about 50 mg per kg per day, preferably from about 15 to about 30 mg per kg per day, in single or divided doses. The compounds of this invention may also be administered orally at doses of from about 15 mg/kg to 50 mg/kg or subcutaneously at doses of about 15 mg/kg to 30 mg/kg.

The compounds of this invention elicit an antibacterial effect against a wide variety of bacterial species, but are generally more active against strains of Gram-positive bacteria. Exemplary of the bacteria against which the compounds of this invention are active are various strains of Staphylococci and Streptococci.

The compounds of the invention have enhanced (IV) serum levels and a similar spectrum of antibacterial activity to that of the corresponding parent 16-membered macrolide having a free C-3 hydroxyl group. The compounds are also active against erythromycin-resistant strains of Staphylococci and methicillin-susceptible and methicillin-resistant strains of Staphylococci.

Specifically, O-(α-L-cladinosyl)-(1→3)-12,13-dihydro-12,13-deoxorosaramicin, a compound of this invention compared to 12,13-dehydro-12,13-deoxorosaramicin is slightly less active against erythromycin-resistant strains of Staphylococci and against sensitive Gram-positive bacteria, but equal to, or slightly more active against various strains of Streptococci.

The antibacterial activity of the compounds of this invention is determined by testing against a variety of pathogens using standard antibacterial dilution assays in Mueller-Hinton agar, the activity being expressed as the Minimum Inhibitory Concentration (MIC, mcg./ml., 24 hours). The geometric mean MIC'S of the compounds of this invention are in the range of about 0.088 to 27.

Most importantly, the compounds of this invention are active antibacterial agents which afford good serum levels at the antibacterially effective dosages provided hereinabove.

## EXAMPLE 1 (starting materials)

### 4-O-Acetyl-1-Deoxy-1-(Pyridyl-2-thio)-α-and -β-L-Cladinoside

#### (a) L-Cladinose

Erythromycin A (Merck Index, 10th Edition Entry No. 3624) (50g) was dissolved in 1N aqueous hydrochloric acid (70 mL) and water (500 mL) and the mixture subjected to liquid-liquid extraction using diethyl ether for 99 h. The ether extract was evaporated to dryness to give L-cladinose (11.7g, 97%) as a colorless viscous oil.

#### (b) 1,4-Di-O-acetyl-β-L-cladinose

L-Cladinose (16.23g) and acetic anhydride (66.7 mL) were dissolved in dry pyridine (133 mL) and the mixture was allowed to remain at 25°C for 18 h. The mixture was evaporated to dryness and the residue was azeotroped with toluene. The product was chromatographed on a silica gel column (45x5 cm) using 3% ethyl acetate in chloroform (v/v) as the eluant to give 1,4-di-O-acetyl-β-L-cladinose (19.2g, 80%) as a colorless solid (Anal. found: C,55.79; H, 7.91%; $C_{12}H_{20}O_6$ requires: C, 55.37; H, 7.74%); MS: 201 ($MH^+$-$CH_3COO$); Rotation: $[\alpha]_D^{26}$ -41.3°($CHCl_3$); IR: $\nu_{max}$ ($CDCl_3$) 1750, 1735, 1238, 1050 $cm^{-1}$; $^1$H-NMR: $\delta_H$ -($CDCl_3$) 1.17 (3H,d,$J_{5ax,6\text{-}CH_3}$7Hz, 6-$CH_3$), 1.17 (3H,s,3-$CH_3$), 1.57 (1H,dd,$J_{1ax,2ax}$ 10Hz, $J_{2ax,2eq}$ 13Hz $H_{2ax}$), 2.12 (3H,s,1-$OCOCH_3$), 2.15 (3H,s,4-$OCOCH_3$), 2.28 (1H,dd,$J_{1ax,2eq}$ 2Hz,$J_{2ax,2eq}$ 13Hz,$H_{2eq}$ 3.32 (3H,s,3-$OCH_3$), 4.10 (1H,dq,$J_{5ax,6\text{-}CH_3}$ 7Hz, $J_{4ax,5ax}$ 10Hz, $H_{5ax}$), 4.68 (1H,d,$J_{4ax,5ax}$ 10Hz,$H_{4ax}$) and 5.92 (1H,dd,$J_{1ax,2ax}$ 10Hz, $J_{1ax,2eq}$ 2Hz, $H_{1ax}$).

(c) 4-O-Acetyl-α-and -β-L-cladinose

1,4-Di-O-actyl-β-L-cladinose (2.18g) was dissolved in 0.1N aqueous hydrochloric acid (245 mL) and the mixture was allowed to remain at 25°C for 1 h. The mixture was extracted with diethyl ether and the ether layer was retained. The aqueous acid layer was neutralized and extracted with dichloromethane. The latter was combined with the ether extract and dried ($MgSO_4$), filtered and evaporated to dryness to give 4-O-acetyl-α-and β-L-cladinose (1.86g, 100%) as a colorless gum that was used without further purification.

(d) 4-O-Acetyl-1-deoxy-1-(pyridyl-2-thio)-α-and-β-L-cladinoside

4-O-Acetyl-L-cladinose (1.84g) was dissolved in dry dichloromethane (25 mL) and the solution was cooled to 13°C. After 30 minutes a chilled solution of 2,2′-di-pyridyldisulphide (Aldrithiol-2) (2.8g) and tri-n-butylphosphine (3.45g) in dry dichloromethane (30 mL) was added in one portion. The mixture was allowed to remain at 13°C under dry argon for 19 h. The solution was evaporated to dryness and the residue chromatographed twice on silica gel columns (20x5 cm) using first 4% ethyl acetate in chloroform and then 2% ethyl acetate in chloroform (v/v) as the eluant to give 4-O-acetyl-1-deoxy-1-(pyridyl-2-thio)-α-L-cladinoside (604 mg, 23%) as a colorless gum (Anal.: found: C, 58.15; H, 6.84; N, 4.54% $C_{15}H_{21}NO_4S$ requires: C, 57.86; H, 6.80; N, 4.50%); MS: m/z 312 ($MH^+$); Rotation: $[\alpha]_D^{26}$ -339.0°($CHCl_3$); UV: $\lambda_{max}$ -($CF_3CH_2OH$) 239 nm (ε9,029), 282 nm (ε5,122); IR: $\nu_{max}$ ($CDCl_3$) 1730, 1580, 1418, 1238, 1063, 1045 $cm^{-1}$; $^1$H-NMR: $\delta_H$($CDCl_3$) 1.12 (3H,d,$J_{5ax,6\text{-}CH_3}$ 7Hz, 6-$CH_3$), 1.20 (3H,s,3-$CH_3$), 2.15 (1H,dd,$J_{1eq,2ax}$ 5Hz, $J_{2eq,2ax}$15Hz,$H_{2ax}$), 2.17 (3H,s,4-$OCOCH_3$), 2.58 (1H,dd,$J_{1eq,2eq}$ 2Hz, $J_{2eq,2ax}$ 15Hz, $H_{2eq}$), 3.37 (3H,s,3-$OCH_3$), 4.53 (1H,dq,$J_{5ax,6\text{-}CH_3}$ 7Hz, $J_{4ax,5ax}$ 10Hz, $H_{5ax}$) 4.74 (1H,d,$J_{4ax,5ax}$ 10Hz, $H_{4ax}$), 6.38 (1H, dd, $J_{1eq,2eq}$ 2Hz, $J_{1eq,2ax}$ 5Hz, $H_{1eq}$), 7.00 (1H, m, 1-S-$C_5H_4N(H_5)$), 7.33 (1H,m, 1-S-$C_5H_4N(H_3)$), 7.52 (1H, m, 1-S-$C_5H_4(H_4)$) and 8.51 (1H, m, 1-S-$C_5H_4N(H_6)$), and 4-O-acetyl-1-deoxy-1-(pyridyl-2-thio)-β-L-cladinoside (12) (1.02g, 39%) as a colourless gum (Anal.: found: C, 57.46; H, 6.86; N, 4.36. $C_{15}H_{21}NO_4S$ requires: C, 57.86; H, 6.80; N, 4.50%); MS: m/z 312 ($MH^+$); Rotation: $[\alpha]_D^{26}$ -1.1° ($CHCl_3$); UV: $\lambda_{max}$ ($CF_3CH_2OH$) 237 nm (ε7,814), 279 nm (ε4,585); IR: $\nu_{max}$ ($CDCl_3$) 1737, 1580, 1418, 1238, 1072, 1046 $cm^{-1}$; $^1$H-NMR: $\delta_H$ ($CDCl_3$) 1.16 (3H,d, $J_{5ax,6\text{-}CH_3}$ 7Hz,6-$CH_3$), 1.19 (3H, s, 3-$CH_3$), 1.81 (1H, dd, $J_{1ax,2ax}$ 11Hz, $J_{2eq,2ax}$ 16Hz, $H_{2ax}$), 2.16 (3H, s, 4-$OCOCH_3$), 2.44(1H, dd, $J_{1eq,2eq}$ 2Hz, $J_{2eq,2ax}$ 16Hz, $H_{2eq}$), 3.34 (3H, s, 3-$OCH_3$), 4.12 (1H, dq, $J_{5ax,6\text{-}CH_3}$ 7Hz, $J_{4ax,5ax}$ 10Hz, $H_{5ax}$), 4.73 (1H, d, $J_{4ax,5ax}$, 10Hz, $H_{4ax}$), 5.70 (1H, dd, $J_{1ax,2ax}$ 11Hz, $J_{1ax,2eq}$ 2Hz, $H_{1ax}$), 7.06 (1H, m, 1-S-$C_5H_4N(H_5)$), 7.35 (1H, m, 1-S-$C_5H_4N(H_3)$), 7.56 (1H, m, 1-S-$C_5H_4N(H_4)$) and 8.42 (1H, m, 1-S-$C_5H_4N(H_6)$).

EXAMPLE 2

O-α-L-Cladinosyl-(1→3)-12,13-dehydro-12,13-deoxorosaramicin

(a) 2′-O-Acetyl-12,13-dehydro-12,13-deoxorosaramicin

12,13-Dehydro-12,13-deoxorosaramicin (2g) and acetic anhydride (1 mL) were dissolved in dry acetone (100 mL) and the solution so formed was allowed to remain at 25°C for 19 h. The reaction mixture was evaporated to dryness and the residue was taken up in dichloromethane and washed with water having a pH equal to 10. The organic layer was dried ($MgSO_4$), filtered and evaporated to dryness. The residue was chromotographed on a silica gel column (60x2 cm) using 10% acetone in chloroform as the eluant to give 2′-O-acetyl-12,13-dehydro-12,13-deoxorosaramicin (1.79g, 83%) as a colorless amorphous solid (Anal.: found: C, 62.06; H, 8.34; N, 1.54%; $C_{33}H_{53}NO_9$ 0.2 $CHCl_3$ requires: C, 62.75; H, 8.46; N, 2.22%); MS: m/z 608 ($MH^+$); Rotation: $[\alpha]_D^{26}$ -4.4°($CHCl_3$; UV: $\lambda_{max}$ ($CF_3CH_2OH$) 288 nm (ε19,165); IR: $\nu_{max}$ ($CDCl_3$) 3530, 1740, 1723, 1675, 1593, 1248, 1060 $cm^{-1}$;$^1$H-NMR: $\delta_H$ ($CDCl_3$) 0.93 (3H,t,$J_{16,17\text{-}CH_3}$ 7Hz, 17-$CH_3$), 0.96

(3H,d,J 7Hz, $CH_3$), 1.08 (3H,d,J 7Hz, $CH_3$), 1.20 (3H,d,J,7Hz,$CH_3$), 1.22 (3H,d,J,7Hz,$CH_3$), 1.81 (3H,d,$J_{12-CH_3,13}$ 1Hz, 12-$CH_3$), 2.08 (3H,s,2′-$OCOCH_3$), 2.26 (6H,s,3′-$N(CH_3)_2$), 4.25 (1H,d,$J_{1ax,2'ax}$ 8Hz, $H_1$′ax), 5.66 (1H,dq,$J_{12-CH,13}$ 1Hz, $J_{13,14}$ 10Hz, $H_{13}$), 6.30 (1H,d,$J_{10,11}$ 16Hz, $H_{10}$), 7.31 (1H,d,$J_{10,11}$ 16Hz, $H_{11}$) and 9.70 (1H,s,$H_{20}$).

(b) 2″-O-Acetyl-O-(4′-O-acetyl-α-L-cladinosyl)-(1→3)-12,13-dehydro-12,13-deoxorosaramicin

2′-O-Acetyl-12,13-dehydro-12,13-deoxorosaramicin (1g) and 4-O-acetyl-1-deoxy-1-(pyridyl-2-thio)-α-and-β-L-cladinoside (from Example 1(d)) (2.83g) were dissolved in dry acetonitrile (60 mL) under dry argon. A solution of anhydrous silver perchlorate (2.22g) in dry acetonitrile (61 mL) was added and the mixture was stirred under dry argon at 25°C for 18 h. The mixture was evaporated to dryness and the residue was taken up in dichloromethane and washed with water, the pH being adjusted to 10. The organic layer was dried ($MgSO_4$), filtered and evaporated to dryness. The residue was chromatographed on a silica gel column (30x5 cm) using ethyl acetate as the eluant to give unreacted 2′-O-acetyl-12,13-dehydro-12,13-deoxorosaramicin (460 mg., 46%) and 2″-O-acetyl-O-(4′-O-acetyl-α-L-cladinosyl)-(1→3)-12,13-dehydro-12,13-deoxorosaramicin (718 mg., 54%) as a colorless amorphous solid (Anal.: found: C, 62.30; H, 8.27; N, 1.53%; $C_{43}H_{69}NO_{13}$. 0.2 $CHCl_3$ requires: C, 62.08; H, 8.36; N, 1.68%); MS: m/z 808 ($MH^+$); Rotation: $[\alpha]_D^{26}$ -46.5° ($CHCl_3$); UV: $\lambda_{max}$ ($CF_3CH_2OH$) 287 nm (ε17,964); IR: $\nu_{max}$ ($CDCl_3$) 1737, 1728, 1673, 1590, 1242, 1050 $cm^{-1}$; $^1$H-NMR: $\delta_H$ ($CDCl_3$) 0.89 (3H,t,$J_{16,17-CH_3}$ 7Hz, 17-$CH_3$), 0.94 (3H,d,J 7Hz, $CH_3$), 1.04 (3H,s,3′-$CH_3$), 1.05 (3H,d,J 7Hz,$CH_3$), 1.06 (3H,d,J 7Hz, $CH_3$), 1.18 (3H,d,J 7Hz, $CH_3$), 1.20 (3H,d,J 7Hz, $CH_3$), 1.79 (3H,d,$J_{12-CH_3,13}$ 1Hz, 12-$CH_3$), 2.08 (3H,s,2″-$OCOCH_3$), 2.11 (3H,s,4′-$OCOCH_3$), 2.29 (6H,s,3″-$N(CH_3)_2$), 3.17 (3H,s,3′-$OCH_3$), 4.33 (1H,d,$J_{1''ax,2''ax}$ 8Hz, $H_1$″ax), 4.66 (1H,d,$J_{4'ax,5'ax}$ 10Hz, $H_4$′ax), 4.74 (1H,dd,$J_{1''ax,2''ax}$ 8Hz, $J_{2''ax,3''ax}$ 10Hz, $H_2$″ax), 4.99 (1H,dd,$J_{1'eq,2'eq}$ 1.5Hz, $J_{1'eq,2'ax}$ 4Hz, $H_1$′eq), 5.64 (1H,dq, $J_{12-CH_3,13}$ 1Hz, $J_{13,14}$ 10Hz, $H_{13}$), 6.25 (1H,d,$J_{10,11}$ 16Hz, $H_{10}$), 7.28 (1H,d,$J_{10,11}$ 16Hz, $H_{11}$) and 9.71 (1H,s,$H_{20}$).

(c) O-α-L-Cladinosyl-(1→3)-12,13-dehydro-12,13-deoxorosaramicin

2″-O-Acetyl-O-(4′-O-acetyl-α-L-cladinosyl)-(1→3)-12,13-dehydro-12,13-deoxorosaramicin (567 mg) was dissolved in a 0.57% (w/v) solution of 1,8-diazabicyclo [5.4.0]undec-7-ene (0.1065 mL) in methanol (19 mL) and the solution was stirred at 25°C under argon for 43 h. The mixture was evaporated to dryness and the residue was taken up in dichloromethane and washed with water, the pH being adjusted to 10. The organic layer was dried ($MgSO_4$), filtered and evaporated to dryness. The residue was chromotographed on a silica gel column (15x5 cm) using 2.5% methanol in chloroform as the eluant to give O-α-L-cladinosyl-(1→3)-12,13-dehydro-12,13-deoxorosaramicin (265 mg., 52%) as a colourless amorphous solid (Anal.: found: C, 64.19; H, 9.46; N, 2.01%; $C_{39}H_{65}NO_{11}$ requires: C, 64.71; H, 9.05; N, 1.93%); MS: m/z 724 ($MH^+$); Rotation: $[\alpha]_D^{26}$ -51.0°($CHCl_3$); UV: $\lambda_{max}$ ($CH_3OH$) 282 nm (ε19,623); IR: $\nu_{max}$ ($CDCl_3$) 3540, 3440, 1728, 1674, 1590, 1160, 1050 $cm.^{-1}$; $^1$H-NMR: $\delta_H$ ($CDCl_3$) 0.90 (3H,t,$J_{16,17-CH_3}$ 7Hz, 17-$CH_3$), 1.05 (3H,d,J 7Hz, $CH_3$), 1.11 (3H,d,J 7Hz, $CH_3$), 1.15 (3H,s,3′-$CH_3$), 1.17 (3H,d,J 7Hz, $CH_3$), 1.19 (3H,d,J 7Hz, $CH_3$), 1.20 (3H,d,J 7Hz, $CH_3$), 1.79 (3H,d,$J_{12-CH_3,13}$ 1Hz, 12-$CH_3$), 2.28 (6H,s,3″-$N(CH_3)_2$), 3.12 (3H,s,3′-$OCH_3$), 4.24 (1H,d,$J_{1''ax,2''ax}$ 8Hz, $H_1$″ax), 4.67 (1H,m,$H_{15}$), 4.94 (1H,dd,$J_{1'eq,2'eq}$ 1Hz, $J_{1'eq,2'ax}$ 4Hz, $H_1$′eq), 5.67 (1H,dq,$J_{12-CH_3,13}$ 1Hz, $J_{13,14}$ 10Hz, $H_{13}$), 6.24 (1H,d,$J_{10,11}$ 16Hz, $H_{10}$), 7.33 (1H,d,$J_{10,11}$ 16Hz, $H_{11}$) and 9.75 (1H,s,$H_{20}$).

## EXAMPLE 3

O-α-L-Cladinosyl-(1→3)-12,13-dehydro-12,13,20-dideoxo-20-dihydrorosaramicin

(a) 2'-O-Acetyl-12,13-dehydro-12,13,20-dideoxo-20-dihydrorosaramicin

12,13-Dehydro-12,13,20-dideoxo-20-dihydrorosaramicin (prepared as described in Example 28 of US Patent 4,056,616) (4g) was dissolved in dry acetone (167 mL) and acetic anhydride (2.05 mL) was added. The mixture was stirred at 25°C for 18 h under dry argon. The solution was evaporated to dryness and the residue was taken up in dichloromethane and washed with water, the pH being adjusted to 10. The organic layer was dried ($MgSO_4$), filtered and evaporated to dryness. The residue was chromatographed on a silica gel column (60x2.5 cm) using 10% acetone in chloroform (v/v) as the eluant to give 2'-O-acetyl-12,13-dehydro-12,13,20-dideoxo-20-dihydrorosaramicin (3.7g, 85%) as a colourless amorphous solid (Anal.: found: C, 65.14; H, 9.04; N, 2.19%; $C_{33}H_{55}NO_8$.0.1 $CHCl_3$ requires: C, 65.43; H, 9.15; N, 2.31%); MS: m/z 594 ($MH^+$); Rotation: $[\alpha]_D^{26}$ +7.9° ($CHCl_3$); UV: $\lambda_{max}$ ($CF_3CH_2OH$) 287 nm ($\epsilon$21,705); IR: $\nu_{max}$ ($CDCl_3$) 3310, 1737, 1725, 1677, 1592, 1250, 1188, 1060 $cm^{-1}$; $^1$H-NMR: $\delta_H$ ($CDCl_3$) 0.85 (3H,t,$J_{16,17\text{-}CH_3}$ 7Hz, 17-$CH_3$), 0.94 (3H,t,$J_{19,20\text{-}CH_3}$ 7Hz, 20-$CH_3$), 0.99 (3H,d,J 7Hz, $CH_3$), 1.20 (3H,d,J 7Hz, $CH_3$), 1.08 (3H,d,J 7Hz, $CH_3$), 1.22 (3H,d,J 7Hz, $CH_3$), 1.79 (3H,d,$J_{12\text{-}CH_3,13}$ 1Hz, 12-$CH_3$), 2.09 (3H,s,2'-$OCOCH_3$), 2.27 (6H,s,3'-$N(CH_3)_2$), 3.46 (1H,m,$H_5$'ax), 3.72 (1H,dd,$J_{4,5}$ 10Hz, $J_{5,6}$ 1Hz, $H_5$), 4.33 (1H,d,$J_1$'ax,2'ax 8Hz, $H_1$'ax), 4.70 (1H,m,$H_{15}$), 4.80 (1H,dd,$J_1$'ax,2'ax 8Hz, $J_2$'ax,3'ax 10Hz, $H_2$'ax), 5.64 (1H,dq,$J_{12\text{-}CH_3,13}$ 1Hz, $J_{13,14}$ 10Hz, $H_{13}$), 6.33 (1H,d,$J_{10,11}$ 16Hz, $H_{10}$) and 7.32 (1H,d,$J_{10,11}$ 16Hz, $H_{11}$).

(b) 2''-O-Acetyl-O-(4'-O-acetyl-α-L-cladinosyl)-(1→3)-12,13-dehydro-12,13,20-dideoxo-20-dihydrorosaramicin

2'-O-Acetyl-12,13-dehydro-12,13,20-dideoxo-20-dihydrorosaramicin (1.57g) and 4-O-acetyl-1-deoxy-1-(pyridyl-2-thio)-α and-β-L-cladinoside (Example 1(d)) (4.55g) were dissolved in dry acetonitrile (111 mL) under dry argon. A solution of anhydrous silver perchlorate (3.61g) in dry acetonitrile (100 mL) was added and the mixture was stirred under dry argon at 25°C for 26 h. The solution was evaporated to dryness and the residue was taken up in dichloromethane and washed with water, the pH being adjusted to 10. The organic layer was dried ($MgSO_4$), filtered and evaporated to dryness. The residue was chromatographed on a silica gel column (30x5 cm) using ethyl acetate as the eluant to give unreacted 2'-O-acetyl-12,13-dehydro-12,13,20-dideoxo-20-dihydrorosaramicin (471 mg., 30%) and 2''-O-acetyl-O-(4'-O-acetyl-α-L-cladinosyl)-(1→3)-12,13-dehydro-12,13,20-dideoxo-20-dihydrorosaramicin (1.31g, 63%) as a colorless amorphous solid (Anal.: found: C, 60.51; H, 8.26; N, 1.22%; $C_{43}H_{71}NO_{12}$. 0.5 $CHCl_3$ requires: C, 60.50; H, 8.38; N, 1.64%); MS: m/z 794 ($MH^+$); Rotation: $[\alpha]_D^{26}$ -91.2° ($CHCl_3$); UV: $\lambda_{max}$ ($CF_3CH_2OH$) 286 nm ($\epsilon$19,336), IR: $\nu_{max}$ ($CDCl_3$) 1733, 1673, 1591, 1240, 1160, 1048 $cm^{-1}$; $^1$H-NMR: $\delta_H$ ($CDCl_3$) †0.89 (3H,t,$J_{16,17\text{-}CH_3}$ 7Hz, 17-$CH_3$), 0.90 (3H,t,$J_{19,20\text{-}CH_3}$ 7Hz, 20-$CH_3$), 0.96 (3H,d,$J_{4,4\text{-}CH_3}$ 7Hz, 4-$CH_3$), 1.03 (3H,d,$J_{14,14\text{-}CH_3}$ 7Hz, 14-$CH_3$), 1.04 (3H,s,3'-$CH_3$), 1.09 (3H,d,$J_5$'ax,6'-$CH_3$ 7Hz, 6'-$CH_3$), 1.18 (3H,d,$J_{8,8\text{-}CH_3}$ 7Hz, 8-$CH_3$), 1.18 (3H,d, $J_5$''ax,6''-$CH_3$ 7Hz, 6''-$CH_3$), 1.78 (3H,d,$J_{12\text{-}CH_3,13}$ 1Hz, 12-$CH_3$), 2.08 (3H,s,2''-$OCOCH_3$), 2.12 (3H,s,4'-$OCOCH_3$), 2.28 (6H,s,3''-$N(CH_3)_2$), 3.19 (3H,s,3'-$OCH_3$), 3.51 (1H,m,$H_5$''ax), 3.69 (1H,dd,$J_{4,5}$ 10Hz, $J_{5,6}$ 1Hz, $H_5$), 4.36 (1H,d,$J_1$''ax,2''ax 8Hz, $H_1$''ax), 4.63 (1H,m,$H_{15}$), 4.65 (1H,d,$J_4$'ax,5'ax 10Hz, $H_4$'ax), 4.74 (1H,dd,$J_1$''ax,2''ax 8Hz, $J_2$''ax,3''ax 10Hz, $H_2$''ax), 4.85 (1H,dd,$J_1$'eq,2'eq 1.5Hz, $J_1$'eq,2'ax 4Hz, $H_1$'eq), 5.59 (1H,dq,$J_{12\text{-}CH_3,13}$ 1Hz, $J_{13,14}$ 10Hz, $H_{13}$), 6.23 (1H,d,$J_{10,11}$ 16Hz, $H_{10}$) and 7.23 (1H,d,$J_{10,11}$ 16Hz, $H_{11}$).

(c) O-α-L-Cladinosyl-(1→3)-12,13-dehydro-12,13,20-dideoxo-20-dihydrorosaramicin

2''-O-Acetyl-O-(4'-O-acetyl-α-L-cladinosyl-(1→3)-12,13-dehydro-12,13,20-dideoxo-20-dihydrorosaramicin (750 mg) and 1,8-diazabicyclo[5.4.0]undec-7-ene (0.705 mL) were dissolved in methanol (25 mL) and the solution was stirred at 25°C for 20 h. The mixture was evaporated to dryness and the residue was taken up in dichloromethane and washed with water, the pH being adjusted to 10. The organic layer was dried ($MgSO_4$), filtered and evaporated to dryness. The residue was chromatographed on a silica gel column (60x2 cm) using 2% methanol in chloroform (v/v) as the eluant. The product was rechromatographed on a silica gel column (15x2 cm) using first chloroform (1 L) and then 2.5% increasing to 10% methanol in

†The proton assignments were confirmed by means of a 2D-NMR experiment at 400 MHz ($\delta_H$/J correlation).

chloroform (v/v) as the eluant to give O-α-L-cladinosyl-(1→3)-12,13-dehydro-12,13,20-dideoxo-20-dihydrorosaramicin (428 mg., 64%) as a colorless amorphous solid. (Anal.: found: C, 61.55; H, 8.78; N, 1.56%; $C_{39}H_{67}NO_{10}$. 0.4 $CHCl_3$ requires: C, 61.82; H, 8.91; N, 1.85%); MS: m/z 710 ($MH^+$); Rotation: $[\alpha]_D^{26}$ -47.2° ($CHCl_3$); UV: $\lambda_{max}$ ($CH_3OH$) 282 nm (ε20,594); IR: $\nu_{max}$ ($CDCl_3$) 3550, 3440, 1730, 1670, 1590, 1160, 1055, 1020 $cm^{-1}$; $^1H$-NMR: $\delta_H$ ($CDCl_3$) 0.89 (3H,t,$J_{16,17-CH_3}$ 7HZ, 17-$CH_3$), 0.93 (3H,t,$J_{19,20-CH_3}$, 7Hz, 20-$CH_3$), 1.04 (3H,d,J 7Hz, $CH_3$), 1.13 (3H,d,J 7Hz, $CH_3$), 1.15 (3H,s,3′-$CH_3$), 1.18 (3H,d,J 7Hz, $CH_3$), 1.20 (3H,d,J 7Hz, $CH_3$), 1.24 (3H,d,J 7Hz, $CH_3$), 1.78 (3H,d,$J_{12-CH_3,13}$ 1Hz, 12-$CH_3$), 2.28 (6H,s,3″-$N(CH_3)_2$), 3.14 (3H,s,3′-$OCH_3$), 4.26 (1H,d,$J_{1''ax,2''ax}$ 8Hz, $H_{1''}$ax), 4.64 (1H,m,$H_{15}$), 4.81 (1H,dd,$J_{1'eq,2'eq}$ 1Hz, $J_{1'eq,2'ax}$ 4Hz, $H_{1'}$eq), 5.62 (1H,dq,$J_{12-CH_3,13}$ 1Hz, $J_{13,14}$ 10Hz, $H_{13}$), 6.23 (1H,d,$J_{10,11}$ 16Hz, $H_{10}$) and 7.28 (1H,d,$J_{10,11}$ 16Hz, $H_{11}$).

EXAMPLE 4 (starting materials)

2″,4″,4‴-Tri-O-acetyl-O-(4′-O-acetyl-α-L-cladinosyl)-(1→3)-Desmycosin

(a) 2′,4′,4″-Tri-O-acetyldesmycosin

Desmycosin (Merck Index, Tenth Edition, p. 1405) (3.6g) and acetic anhydride (1.54 mL) were dissolved in dry pyridine (150 mL) and the mixture was allowed to remain at 25°C for 74 h. The mixture was evaporated to dryness and the residue was azeotroped with toluene. The residue was chromatographed on a silica gel column (30x5 cm) using 15% acetone in chloroform as the eluant to give 2′,4′,4″-tri-O-acetyldesmycosin (1.91 g., 46%) as a colorless amorphous solid. (Anal.: found: C, 60.09; H, 8.08; N, 1.44%; $C_{45}H_{71}NO_{17}$ requires: C, 60.18; H, 7.97; N, 1.56%); MS: m/z 898 ($MH^+$); Rotation: $[\alpha]_D^{26}$ -3.1° ($CHCl_3$); UV: $\lambda_{max}$ ($CF_3CH_2OH$) 286 nm (ε22,009); IR: $\nu_{max}$ ($CDCl_3$) 3530, 1740, 1678, 1593, 1235, 1167, 1050 $cm^{-1}$; $^1H$-NMR: $\delta_H$ ($CDCl_3$) 0.93 (3H,t,$J_{16,17-CH_3}$ 7Hz, 17-$CH_3$), 0.97 (3H,d,J 7Hz, $CH_3$), 1.14 (3H,d,J 7Hz, $CH_3$), 1.18 (3H,d,J 7Hz, $CH_3$), 1.22 (3H,d,J 7Hz, $CH_3$), 1.80 (3H,d,$J_{12-CH_3,13}$ 1Hz, 12-$CH_3$), 2.05 (3H,s,4′-$OCOCH_3$), 2.06 (3H,s,2′-$OCOCH_3$) 2.12 (3H,s,4″-$OCOCH_3$), 2.34 (6H,s,3′-$N(CH_3)_2$), 3.50 (3H,s,2″-$OCH_3$), 3.54 (3H,s,3″-$OCH_3$), 4.33 (1H,d,$J_{1'ax,2'ax}$ 8Hz, $H_{1'}$ax), 4.66 (1H,d,$J_{1''ax,2''ax}$ 8Hz, $H_{1''}$ax), 5.94 (1H,dq,$J_{12-CH_3,13}$ 1Hz, $J_{13,14}$ 10Hz, $H_{13}$), 6.32 (1H,d,$J_{10,11}$ 16Hz, $H_{10}$), 7.36 (1H,d,$J_{10,11}$ 16Hz, $H_{11}$), and 9.71 (1H,s,$H_{20}$).

(b) 2″,4″,4‴-Tri-O-acetyl-O-(4′-O-acetyl-α-L-cladinosyl)-(1→3)-desmycosin

2′,4′,4″-Tri-O-acetyldesmycosin (335 mg) and 4-O-acetyl-1-deoxy-1-(pyridyl-2-thio)-α-and -β-L-cladinoside (Example 1(d)) (953.5 mg) were dissolved in dry acetonitrile (20 mL) under dry argon. A solution of anhydrous silver perchlorate (750.5 mg) in dry acetonitrile (20.6 mL) was added and the mixture was stirred under dry argon at 25°C for 23 h. The mixture was evaporated to dryness and the residue was taken up in dichloromethane and washed with water, the pH being adjusted to 10. The organic layer was dried ($MgSO_4$), filtered and evaporated to dryness. The residue was chromatographed on a silica gel column (60x2 cm) using 25% acetone in hexane (v/v) as the eluant. The product was rechromatographed on a silica gel column (60x2 cm) using 15% acetone in chloroform (v/v) as the eluant to give 2″,4″,4‴-tri-O-acetyl-O-(4′-O-acetyl-α-L-cladinosyl)-(1→3)-desmycosin (167 mg., 41%) as a colorless amorphous solid (Anal.: found: C, 60.11; H, 8.11; N, 1.26%; $C_{55}H_{87}NO_{21}$ requires: C, 60.15; H, 7.98; N, 1.28%); MS: m/z 1098 ($MH^+$); Rotation: $[\alpha]_D^{26}$ -35.1° ($CHCl_3$); UV: $\lambda_{max}$ ($CF_3CH_2OH$) 285 nm (ε22,465); IR: $\nu_{max}$ 1740, 1680, 1595, 1240, 1168, 1050 $cm^{-1}$; $^1H$-NMR: $\delta_H$ ($CDCl_3$) 0.90 (3H,t,$J_{16,17-CH_3}$ 7Hz, 17-$CH_3$), 0.91 (3H,d,J 7Hz, $CH_3$), 1.07 (3H,s,3′-$CH_3$), 1.08 (3H,d,J 7Hz, $CH_3$), 1.13 (3H,d,J 7Hz, $CH_3$), 1.16 (3H,d,J 7Hz, $CH_3$), 1.20 (3H,d,J 7Hz, $CH_3$), 1.79 (3H,d,$J_{12-CH_3,13}$ 1Hz, 12-$CH_3$), 2.04 (3H,s,4″-$OCOCH_3$), 2.05 (3H,s,2″-$OCOCH_3$), 2.12 (6H,s,4′-$OCOCH_3$ and 4‴-$OCOCH_3$), 2.37 (6H,s,3″-$N(CH_3)_2$), 3.19 (3H,s,3′-$OCH_3$), 3.47 (3H,s,2‴-$OCH_3$), 3.53 ( 3H,s,3‴-$OCH_3$), 4.46 (1H,d,$J_{1''ax,2''ax}$ 8Hz, $H_{1''}$ax), 4.62 (1H,d,$J_{1'''ax,2'''ax}$ 8Hz, $H_{1'''}$ax), 5.00 (1H,dd,$J_{1'eq,2'eq}$ 1Hz, $J_{1'eq,2'ax}$ 4Hz, $H_{1'}$eq), 5.88 (1H,dq,$J_{12-CH_3,13}$ 1Hz, $J_{13,14}$ 10Hz, $H_{13}$), 6.25 (1H,d,$J_{10,11}$ 16Hz, $H_{10}$), 7.31 (1H,d,$J_{10,11}$ 16Hz, $H_{11}$), and 9.70 (1H,s,$H_{20}$).

EXAMPLE 5

2″,4″,4‴-Tri-O-Acetyl-O-(4′-O-phenoxyacetyl-α-L-cladinosyl)-(1→3)-desycosin

(a) 1,4-Di-O-phenoxyacetyl-β-L-cladinose

(i) L-Cladinose (Example 1(a)) (2.85 g) and pyridine (19.65 mL) were dissolved in dry dichloromethane (84 mL) and the solution was cooled to 0°C under argon. A solution of phenoxyacetyl chloride (11.2 mL) in dry dichloromethane (84 mL) was added dropwise to the stirred solution over 1 h. The mixture was stirred at 0°C for 2.5 h. The mixture was diluted with dichloromethane and the washed with water, the pH being adjusted to 10. The organic layer was dried ($MgSO_4$), filtered and evaporated to dryness. The residue was azeotroped with toluene and chromatographed on a silica gel column (30x5 cm) using 1.5% acetone in chloroform (v/v) as the eluant to give 1,4-di-O-phenoxyacetyl-β-L-cladinose (3.3g, 46%) as a colourless gum (Anal.: found: C, 61.97; H, 6.20%; $C_{24}H_{28}O_8$ requires: C, 64.85; H, 6.35%); MS: m/z 293 ($MH^+$-$OCOCH_2OC_6H_5$); Rotation: $[\alpha]_D^{26}$ -26.0° ($CHCl_3$); UV: $\lambda_{max}$ ($CH_3OH$) 206 nm (ε13,285), 260 nm (ε1,418), 266 nm (ε2,012), 273 nm (ε1,601); IR: $\nu_{max}$ ($CDCl_3$) 1775, 1598, 1590, 1490, 1273, 1075, 1030 $cm^{-1}$; $^1$H-NMR: $\delta_H$ ($CDCl_3$) 1.17 (3H,s,3-$CH_3$), 1.18 (3H,d,$J_{5ax,6\text{-}CH_3}$ 7Hz, 6-$CH_3$), 1.63 (1H,dd,$J_{1ax,2ax}$ 10Hz, $J_{2eq,2ax}$ 15Hz, $H_{2ax}$), 2.34 (1H,dd,$J_{1ax,2eq}$ 4Hz, $J_{2eq,2ax}$ 15Hz, $H_{2eq}$), 3.31 (3H,s,3-$OCH_3$), 4.20 (1H,dq,$J_{4ax,5ax}$ 10Hz, $J_{5ax,6\text{-}CH_3}$ 7Hz, $H_{5ax}$), 4.66 (1H,d,J 16Hz, 1-$OCOCH_2OC_6H_5$), 4.71 (1H,d,J 16Hz, 1-$OCOCH_2OC_6H_5$), 4.73 (1H,d,J 16Hz, 4-$OCOCH_2OC_6H_5$), 4.78 (1H,d,J 16Hz, 4-$OCOCH_2OC_6H_5$), 4.82 (1H,d,$J_{4ax,5ax}$ 10Hz, $H_{4ax}$), 6.08 (1H,dd,$J_{1ax,2eq}$ 4Hz, $J_{1ax,2ax}$ 10Hz, $H_{1ax}$), 6.94 (4H,d,J 9Hz, 1-and 4-$OCOCH_2OC_6H_5$($H_2$ and $H_6$)), 7.05 (2H,dd,J 9Hz, J 9Hz, 1-and 4-$OCOCH_2OC_6H_5$ ($H_4$)) and 7.35 (4H,dd,J 9Hz, J 9Hz, 1-and 4-$OCOCH_2OC_6H_5$ ($H_3$ and $H_5$)).

(ii) L-Cladinose(10.82g) and pyridine (75.2 mL) were dissolved in dry dichloromethane (160 mL). A solution of phenoxyacetic anhydride (87.9 g) in dry dichloromethane (160 mL) was added dropwise to the stirred solution at 25°C over 30 m. The mixture was diluted with dichloromethane and washed with water, the pH being adjusted to 10. The organic layer was dried ($MgSO_4$), filtered and evaporated to dryness. The residue was azeotroped with toluene and chromatographed by preparative HPLC on two silica gel cartridges using dichloromethane as the eluant to give 1,4-di-O-phenoxyacetyl-β-L-cladinose (17) (21.75g., 80%) which was identical with that prepared in 5(a) (i) above.

(b) 4-O-Phenoxyacetyl-α-and-β-L-cladinose

1,4-Di-O-phenoxyacetyl-β-L-cladinose (63.35 g) was dissolved in acetonitrile (4.5 L) and 0.1N hydrochloric acid (4.158 L) was added and the mixture was stirred at 25°C for 18 h. The reaction mixture was neutralized to pH 7.3 and the acetonitrile was evaporated *in vacuo.* The resulting aqueous solution was extracted with diethyl ether (2x1.5 L) and then with dichloromethane (2x1.5 L). The combined organic extracts were evaporated to dryness and the residue was chromotographed on a preparative HPLC using two silica gel cartridges. The material was eluted using first dichloromethane and then 5% acetone in dichloromethane (v/v) to give 4-O-phenoxyacetyl-α-and β-L-cladinose (32 g., 72%) as a colorless gum that was used directly in the following experiment.

(c) 1-Deoxy-4-O-phenoxyacetyl-1-(pyridyl-2-thio-α-and -β-L-cladinoside

4-O-Phenoxyacetyl-α-and -β-L-cladinose (32g) were dissolved in dry dichloromethane (288 mL) and the solution was cooled to 14°C. A chilled solution of 2,2′-dipyridyldisulfide (Available from Aldrich Chemical Co. as Aldrithiol-2) (29.53g) and tri-n-butylphosphine (36.3 mL) in dry dichloromethane (288 mL) was added in one portion. The mixture was allowed to remain at 14°C under dry argon for 20 h. The mixture was washed with water, the pH being adjusted to 10. The organic layer was dried ($MgSO_4$), filtered and evaporated to dryness. The residue was chromatographed on a preparative HPLC using two silica gel cartridges. The material was eluted first with hexane (4 L.), then with 25% dichloromethane in hexane (4 L.), dichloromethane (4 L.) and finally 2% acetone in dichlormethane (4 L.) (v/v), to give a mixture of 1-deoxy-4-O-phenoxyacetyl-1-(pyridyl-2-thio)-α-and β-L-cladinoside (36.7 g., 88%) (49% α; 39% β).

An aliquot was rechromatographed on a silica gel column (20x5 cm) using dichloromethane and then 0.5% acetone in dichlormethane (v/v) as the eluant to give from the more polar fractions, the pure β-anomer as a colorless solid; m.p. 74.5-79°C, (Anal.: found: C, 62.28; H, 6.26; N, 3.82; S, 7.72%. $C_{21}H_{25}NO_5S$ requires: C, 62.51; H, 6.25; N, 3.47; S, 7.95%); MS: m/z 404 ($MH^+$); Rotation: [ $\alpha]_D^{26}$ +13.9° ($CHCl_3$); UV: $\lambda_{max}$ ($CF_3CH_2OH$) 237 nm (ε7,991), 268 nm (ε4,479), 274 nm (ε5,258), 280 nm (ε4,883); IR: $\nu_{max}$ ($CDCl_3$) 1760, 1734, 1596, 1572, 1190, 1065, 1005 $cm^{-1}$; $^1$H-NMR: $\delta_H$ ($CDCl_3$) 1.16 (3H,d,$J_{5ax,6-}$ $CH_3$ 7Hz, 6-$CH_3$), 1.18 (3H,s,3-$CH_3$), 1.86 (1H,dd,$J_{1ax,2ax}$ 12Hz, $J_{2eq,2ax}$ 15Hz, $H_{2ax}$), 2.44 (1H,dd,$J_{1ax,2eq}$ 3Hz, $J_{2eq,2ax}$ 15Hz, $H_{2eq}$), 3.32 (3H,s,3-$OCH_3$), 4.18 (1H,dq,$J_{5ax,6-CH_3}$ 7Hz, $J_{4ax,5ax}$ 10Hz, $H_{5ax}$), 4.74 (1H,d,J 16Hz, 4-$OCOC\underline{H}_2OC_6H_5$), 4.78 (1H,d,J 16Hz, 4-$OCOC\underline{H}_2OC_6H_5$), 4.86 (1H,d,$J_{4ax,5ax}$ 10Hz, $H_{4ax}$), 5.74 (1H,dd,$J_{1ax,2eq}$ 3Hz, $J_{1ax,2ax}$ 12Hz, $H_{1ax}$), 6.94 (2H,d,J 9Hz, 4-$OCOCH_2OC_6\underline{H}_5$ ($H_2$ and $H_5$)), 7.03 (1H,dd,J 9Hz, J 9Hz, 4-$OCOCH_2OC_6\underline{H}_5$ ($H_4$), 7.03 (1H,m,1-S-$C_4\underline{H}_4N(H_5)$), 7.32 (2H,dd,J 9Hz, J 9Hz, 4-$OCOCH_2OC_6\underline{H}_5$ ($C_2$ and $C_5$)), 7.35 (1H,m,1-S-$C_5\underline{H}_4N(H_3)$), 7.58 (1H,ddd,$J_{3,4}=J_{4,5}=$9Hz, $J_{4,6}$ 2.5Hz, 1-S-$C_5\underline{H}_4N(H_4)$) and 8.48 (1H,m,1-S-$C_5\underline{H}_4N(H_6)$). The less polar fractions were combined and rechromatographed on a silica gel column (15x5 cm) using 2.5% acetone in hexane as the eluant to give the pure α-anomer as a colorless solid, m.p. 131-133.5°C, (Anal.: found: C, 62.69; H, 6.29; N, 3.32; S, 8.18%. $C_{21}H_{25}NO_5S$ requires: C, 62.51; H, 6.25; N, 3.47; S, 7.95%), MS: m/z 404 ($MH^+$); Rotation: $[\alpha]_D^{26}$ -272.1° ($CHCl_3$); UV: $\lambda_{max}$ ($CF_3CH_2OH$) 237 nm (ε15,737), 268 nm (ε8,675), 274 nm (ε10,067), 281 nm (ε9,180); IR: $\nu_{max}$ ($CDCl_3$) 1760, 1595, 1575, 1190, 1063 $cm^{-1}$; $^1$H-NMR; $\delta_H$ ($CDCl_3$) 1.13 (3H,d,$J_{5ax,6-CH_3}$ 7Hz, 6-$CH_3$), 1.18 (3H,s,3-$CH_3$), 2.21 (1H,dd,$J_{1eq,2ax}$ 6Hz, $J_{2eq,2ax}$ 15Hz, $H_{2ax}$), 2.50 (1H,dd,$J_{1eq,2eq}$ 1Hz, $J_{2eq,2ax}$ 15Hz, $H_{2eq}$), 3.34 (3H,s,3-$OCH_3$), 4.53 (1H,dq,$J_{4ax,5ax}$ 10Hz, $J_{5ax,6-CH_3}$ 7Hz, $H_{5ax}$), 4.75 (1H,d,J 16Hz, 4-$OCOC\underline{H}_2OC_6H_5$, 4.79 (1H,d,J 16Hz, 4-$OCOC\underline{H}_2OC_6H_5$), 4.86 (1H,d,$J_{4ax,5ax}$ 10Hz, $H_{4ax}$), 6.44 (1H,dd,$J_{1eq,2eq}$ 1Hz, $J_{1eq,2ax}$ 6Hz, $H_{1eq}$), 6.96 (2H,d,J 9Hz, 4-$OCOCH_2OC_6\underline{H}_5$ ($H_2$ and $H_6$)), 7.06 (1H,dd,J 9Hz J 9Hz, 4-$OCOCH_2OC_6\underline{H}_5$ ($H_4$)), 7.06 (1H,m,1-S-$C_5\underline{H}_4N(H_5)$), 7.34 (2H,dd,J 9Hz, J9Hz, 4-$OCOCH_2OC_6\underline{H}_5$ ($H_3$ and $H_5$)), 7.34 (1H,m,1-S-$C_5\underline{H}_4N(H_3)$), 7.56 (1H,ddd,$J_{3,4}=J_{4,5}=$9Hz, $J_{4,6}$ 2.5Hz, 1-S-$C_5\underline{H}_4N(H_4)$) and 8.52 (1H,m,1-S-$C_5\underline{H}_4N(H_6)$).

(d) 2″,4″,4‴-Tri-O-acetyl-O-(4′-O-phenoxyacetyl-α-L-cladinosyl)-(1→3)-desmycosin

2′4′,4″-Tri-O-acetyldesmycosin (Example 4(a)) (2.5 g.) and 1-deoxy-4-O-phenoxyacetyl-1-(pyridyl-2-thio)-α-and-β-L-cladinoside (Example 5(c)) (6.2 g.) were added to a solution of anhydrous silver trifluoromethanesulphonate (4.63 g) in dry acetonitrile (100 mL) and the mixture was stirred under dry argon at 25°C for 20 h. The mixture was evaporated to dryness and the residue was taken up in dichlormethane and washed with water, the pH being adjusted to 10. The organic layer was dried ($MgSO_4$), filtered and evaporated to dryness. The residue was chromatographed on a silica gel column (15x5 cm) using 11% acetone in hexane (v/v) as the eluant to give two partially purified cuts of the product. Each was rechromatographed on a silica gel column (30x2.5 cm using 20% ethyl acetate in dichloromethane (v/v) to give a combined pure sample of 2″,4″,4‴-tri-O-acetyl-O-(4′-O-phenoxyacetyl-α-L-cladinosyl)-(1→3)-desmycosin (1.99 g., 60%) as a colorless amorphous solid (Anal.: found: C, 61.34; H, 7.84; N, 0.88%; $C_{61}H_{91}NO_{22}$ requires: C, 61.55; H, 7.71; N, 1.18%), MS: m/z 1190 ($MH^+$); Rotation: $[\alpha]_D^{26}$ -30.0° ($CHCl_3$); UV: $\lambda_{max}$ ($CF_3CH_2OH$) 284 nm (ε22,451), IR: $\nu_{max}$ ($CDCl_3$) 1740, 1675, 1590, 1230, 1168, 1045 $cm^{-1}$; $^1$H-NMR: $\delta_H$ ($CDCl_3$), 0.89 (3H,t,$J_{16,17-CH_3}$ 7Hz, 17-$CH_3$), 0.92 (3H,d,J 7Hz, $CH_3$), 1.05 (3H,s,3′-$CH_3$), 1.10 (3H,d,J 7Hz, $CH_3$), 1.12 (3H,d,J 7Hz, $CH_3$), 1.17 (3H,d,J 7Hz, $CH_3$), 1.21 (3H,d,J 7Hz, $CH_3$), 1.80 (3H,d,$J_{12-CH_3,13}$ 1Hz, 12-$CH_3$), 2.02 (3H,s,4″-$OCOCH_3$), 2.06 (3H,s,2″-$OCOCH_3$), 2.13 (3H,s,4‴-$OCOCH_3$), 2.35 (6H,s,3″-$N(CH_3)_2$), 3.19 (3H,s,3′-$OCH_3$), 3.46 (3H,s,2‴-$OCH_3$), 3.52 (3H,s,3‴-$OCH_3$), 4.30 (1H,d,$J_1$″ax,2″ax 8Hz, $H_1$″ax), 4.62 (3H,d,$J_1$‴ax,2‴ax 8Hz, $H_1$‴ax), 4.65 (1H,d,J 16Hz, 4′-$OCOC_{H2}OC_6H_5$), 4.71 (1H,d,J 16Hz, 4′-$OCOC\underline{H}_2OC_6H_5$), 4.89 (1H,m,$H_{15}$), 5.02 (1H,dd,$J_1$′eq,2′eq 1Hz, $J_1$′eq,2′ax 4Hz, $H_1$′eq), 5.86 (1H,dq,$J_{12-CH_3,13}$ 1Hz, $J_{13,14}$ 10Hz, $H_{13}$), 6.25 (1H,d,$J_{10,11}$ 16Hz, $H_{10}$), 6.90 (2H,d,J 9Hz, 4′-$OCOCH_2OC_6H_5$ ($H_2$ and $H_6$)), 6.98 (1H,dd,J 9Hz, J 9Hz 4′-$OCOCH_2OC_6\underline{H}_5$ ($H_4$)), 7.29 (2H,dd,J 9Hz, J 9Hz, 4′-$OCOCH_2OC_6\underline{H}_5$ ($H_3$ and $H_5$)), 7.30 (3H,d,$J_{10,11}$ 16Hz, $H_{11}$), and 9.67 (1H,s,$H_{20}$). The more polar fractions afforded some unreacted 2′,4′,4″-tri-O-acetyldesmycosin (190 mg., 8%).

EXAMPLE 6

O-α-L-Cladinosyl-(1→3)-desmycosin

(a) 2′,4′,4″-Tri-O-acetyldesmycosin (Example 4(a)) (800 mg.) and 4-O-acetyl-1-deoxy-1-(pyridyl-2-thio)-α-and β-L-cladinoside (Example 1(d)) (1.53 g) were dissolved in dry acetonitrile (32 mL) under dry argon. A solution of anhydrous silver perchlorate (1.2 g.) in dry acetonitrile (33.1 mL) was added and the mixture was stirred under dry argon at 25°C for 21 h. The mixture was evaporated to dryness and the residue was taken up in dichlormethane and washed with water, the pH being adjusted to 10. The organic layer was dried ($MgSO_4$), filtered and evaporated to dryness. The residue was chromatographed on a silica gel column (15x5 cm.) using 20% acetone in hexane (v/v) as the eluant to give 2″,4″,4‴-tri-O-acetyl-O-(4′-O-acetyl-α-L-cladinosyl)-(1→3)-desmycosin (602 mg). The latter was dissolved in methanol (14.6 mL) containing 1,8-diazabicyclo[5.4.0]undec-7-ene (0.2768 mL) and the mixture was stirred under dry argon at 25°C for 44 h. The mixture was evaporated to dryness and the residue was chromatographed on a silica gel column (30x2 cm) using 3% methanol in chloroform (v/v) as the eluant to give O-α-L-cladinosyl-(1→3)-desmycosin (272 mg., 33%) contaminated with about 5-10% of O-α-L-cladinosyl-(1→3)-desmycosin seco acid methyl ester, which cochromatographed with the desired product.

(b) 2″,4″,4‴-Tri-O-acetyl-O-(4-O-phenoxyacetyl-α-L-cladinosyl)-(1→3)-desmycosin (Example 5(d)) (1.58 g.) was dissolved in methanol (186 mL) containing triethylamine (3.72 mL) (2% w/v solution) and the mixture was allowed to remain under dry argon at 25°C for 70 h. The solution was evaporated to dryness and the residue was chromatographed on a silica gel column (60x2.5 cm.) using 4% methanol in chloroform (v/v) as the eluant to give O-α-L-cladinosyl-(1→3)-desmycosin (1.13g., 91%) as a colorless amorphous solid (Anal.: found: C, 59.15; H, 8.22; N, 1.44. $C_{47}H_{79}NO_{17}$ 0.2 $CHCl_3$ requires: C, 59.17; H, 8.35; N, 1.47%); MS: m/z 930 ($MH^+$); Rotation: $[\alpha]_D^{26}$ -43.8° ($CHCl_3$); UV: $\lambda_{max}$ ($CH_3OH$) 283 nm (ε20,395); IR: $\nu_{max}$ ($CDCl_3$) 3592, 3550, 1737, 1729, 1674, 1590, 1160, 1057 $cm^{-1}$; $^1$H-NMR: $\delta_H$ ($CDCl_3$) 0.89 (3H,t,$J_{16,17\text{-}CH_3}$ 7Hz, 17-$CH_3$), 1.02 (3H,d,J 7Hz, $CH_3$), 1.16 (3H,s,3′-$CH_3$), 1.20 (3H,d,J 7Hz, $CH_3$), 1.21 (3H,d,J 7Hz, $CH_3$), 1.26 (3H,d,J 7Hz, $CH_3$), 1.27 (3H,d,J 7Hz, $CH_3$), 1.79 (3H,d,$J_{12\text{-}CH_3,13}$ 1Hz, 12-$CH_3$), 2.52 (6H,s,3″-N($CH_3$)$_2$), 3.12 3H,s,3′-$OCH_3$), 3.46 (3H,s,2‴-$OCH_3$), 3.60 (3H,s,3‴-$OCH_3$), 4.29 (1H,d,$J_{1''ax,2''ax}$ 8Hz, $H_{1''ax}$), 4.54 (1H,d, $J_{1'''ax,2'''ax}$ 8Hz, $H_{1'''ax}$), 4.88 (1H,m,$H_{15}$), 4.92 (1H,dd,$J_{1'eq,2'eq}$ 1Hz, $J_{1'eq,2'ax}$ 4Hz, $H_{1'eq}$), 5.87 (1H,dq,$J_{12\text{-}CH_3,13}$ 1Hz, $J_{13,14}$ 10Hz, $H_{13}$), 6.23 (1H,d,$J_{10,11}$ 16Hz, $H_{10}$), 7.32 (1H,d,$J_{10,11}$ 16Hz, $H_{11}$), and 9.71 (1H,s,$H_{20}$).

EXAMPLE 7

2″,4″,4‴-Tri-O-acetyl-20-deoxo-20-dihydro-O-(4′-O-phenoxyacetyl-α-L-cladinosyl)-(1→3)-desmycosin

(a) 2′,4′-Di-O-acetyl-20-deoxo-20-dihydrodesmycosin

20-Deoxo-20-dihydrodesmycosin prepared by reduction of desmycosin tosyl hydrazone with bis-(triphenylphosphine) copper (I) tetrahydroborate as described in A.K. Ganguly et al., J. Chem. Soc., Chem. Commun., 1983, 1166 (3g.) and pyridine (3.2 mL were dissolved in dry dichloromethane (430 mL) and acetic anhydride (1.86 mL) was added. The mixture was stirred under dry argon at 25°C for 42 h. The solution was diluted with dichloromethane and washed with water, the pH being adjusted to 10. The organic layer was dried ($MgSO_4$), filtered and evaporated to dryness. The residue was chromatographed on a silica gel column (30x2 cm.) using 10% acetone in chloroform (v/v) as the eluant to give 2′,4′-di-O-acetyl-20-deoxo-20-dihydrodesmycosin (2.92 g., 88%) as a colorless amorphous solid (Anal.: found: C, 60.77; H, 8.26; N, 1.27%; $C_{43}H_{71}NO_{15}$. 0.1 $CHCl_3$ requires: C, 60.47; H, 8.38; N, 1.64%); MS: m/z 842 ($MH^+$); Rotation: $[\alpha]_D^{26}$ -8.2° ($CHCl_3$); UV: $\lambda_{max}$ ($CF_3CH_2OH$) 283 nm (ε22,537); IR: $\nu_{max}$ ($CDCl_3$) 3560, 1744, 1720, 1678, 1596, 1235, 1168, 1057 $cm^{-1}$; $^1$H-NMR: $\delta_H$ ($CDCl_3$) 0.82 (3H,t,$J_{19.20\text{-}CH_3}$ 7Hz, 20-$CH_3$), 0.93 (3H,t,$J_{16,17\text{-}CH_3}$ 7Hz, 17-$CH_3$), 0.95 (3H,d,J 7Hz, $CH_3$), 1.13 (3H,d,J 7Hz, $CH_3$), 1.18 (3H,d,J 7Hz, $CH_3$), 1.26 (3H,d,J 7Hz, $CH_3$), 1.78 (3H,d,$J_{12\text{-}CH_3,13}$ 1Hz, 12-$CH_3$), 2.05 (3H,s,4′-$OCOCH_3$), 2.06 (3H,s,2′-$OCOCH_3$), 2.34 (6H,s,3′-N($CH_3$)$_2$), 3.48 (3H,s,2″-$OCH_3$), 3.61 (3H,s,3″-$OCH_3$), 4.36 (1H,d,$J_{1'ax,2'ax}$ 8Hz, $H_{1'ax}$), 4.55 (1H,d,$J_{1''ax,2''ax}$ 8Hz, $H_{1''ax}$), 5.86 (1H,dq,$J_{12\text{-}CH_3,13}$ 1Hz, $J_{13,14}$ 10Hz, $H_{13}$), 6.28 (1H,d,$J_{10,11}$ 16Hz, $H_{10}$), and 7.29 (1H,d,$J_{10,11}$ 16Hz, $H_{11}$).

(b) 2′,4′-4″-Tri-O-acetyl-20-deoxo-20-dihydrodesmycosin

2′,4′-Di-O-acetyl-20-deoxo-20-dihydrodesmycosin (2.92 g), 4-N,N-dimethylaminopyridine (87 mg) and triethylamine (5 mL) were dissolved in dry dichloromethane (350 mL) and acetic anhydride (0.336 mL) was added. The mixture was stirred under argon at 25°C for 20 h. The reaction mixture was diluted with dichloromethane and washed with water, the pH being adjusted to 10. The organic layer was dried ($MgSO_4$), filtered and evaporated to dryness. The residue was chromatographed on a silica gel column (30x2 cm.) using 7% acetone in chloroform (v/v) as the eluant to give 2′,4′,4″-tri-O-acetyl-20-deoxo-20-dihydrodesmycosin (2.42 g., 79%) as a colourless amorphous solid (Anal: found: C, 60.33; H, 8.09; N, 1.57%; $C_{45}H_{73}NO_{16}$. 0.1 $CHCl_3$ requires: C, 60.32; H, 8.21; N, 1.56%); MS: m/z 884 ($MH^+$); Rotation: $[\alpha]_D^{26}$ +11.2° ($CHCl_3$); UV: $\lambda_{max}$ ($CF_3CH_2OH$) 283 nm (ε21,718), IR: $\nu_{max}$ ($CDCl_3$) 3540, 1742, 1676, 1593, 1238, 1168, 1050 $cm^{-1}$; $^1$H-NMR: $\delta_H$ ($CDCl_3$) 0.83 (3H,t,$J_{19,20\text{-}CH_3}$ 7Hz, 20-$CH_3$), 0.92 (3H,t,$J_{16,17\text{-}CH_3}$ 7Hz, 17-$CH_3$), 0.96 (3H,d,J 7Hz, $CH_3$), 1.13 (3H,d,J 7Hz, $CH_3$), 1.17 (3H,d,J 7Hz, $CH_3$), 1.19 (3H,d,J 7Hz, $CH_3$), 1.79 (3H,d,$J_{12\text{-}CH_3,13}$ 1Hz, 12-$CH_3$), 2.06 (3H,s,4′-$OCOCH_3$), 2.07 (3H,s,2′-$OCOCH_3$), 2.13 (3H,s,4″-$OCOCH_3$), 2.36 (6H,s,3′-$N(CH_3)_2$), 3.49 (3H,s,2″-$OCH_3$), 3.54 (3H,s,3″-$OCH_3$), 4.38 (1H,d,$J_{1'ax,2'ax}$ 8Hz, $H_{1'}$ax), 4.63 (1H,d,$J_{1''ax,2''ax}$ 8Hz, $H_{1''}$ax), 5.88 (1H,dq,$J_{12\text{-}CH_3,13}$ 1Hz, $J_{13,14}$ 10Hz, $H_{13}$), 6.29 (1H,d,$J_{10,11}$ 16Hz, $H_{10}$), and 7.32 (1H,d,$J_{10,11}$ 16Hz, $H_{11}$).

(c) 2″,4″,4‴-Tri-O-acetyl-(4′-O-acetyl-α-L-cladinosyl)-(1→3)-20-deoxo-20-dihydrodesmycosin

2′,4′,4″-Tri-O-acetyl-20-deoxo-20-dihydrodesmycosin (800 mg) and 4-O-acetyl-1-deoxy-1-(pyridyl-2-thio)-α-and -β-L-cladinoside (Example 1(d) (1.55 g.) were dissolved in dry acetonitrile (34 mL) under dry argon. A solution of anhydrous silver perchlorate (1.22 g.) in dry acetonitrile (34 mL) was added and the mixture was stirred under dry argon at 25°C for 19 h. The mixture was evaporated to dryness and the residue was taken up in dichloromethane and washed with water, the pH being adjusted to 10. The organic layer was dried ($MgSO_4$), filtered and evaporated to dryness. The residue was chromatographed on a silica gel column (60x2 cm.) using 15% acetone in hexane (u/v) as the eluant to give 2″,4″,4‴-tri-O-acetyl-(4′-O-acetyl-α-L-cladinosyl)-(1→3)-20-deoxo-20-dihydrodesmycosin (396 mg., 40%) as a colorless amorphous solid; MS: m/z 1082 ($MH^+$).

(d) 2″,4″,4‴-Tri-O-acetyl-20-deoxo-20-dihydro-O-(4′-O-phenoxyacetyl-α-L-cladinosyl)-(1→3)-20-deoxo-desmycosin

2′,4′,4″-Tri-O-acetyl-20-deoxo-20-dihydrodesmycosin (1.83. g.) and 1-deoxy-4-O-phenoxyacetyl-1-(pyridyl-2-thio)-α-and -β-L-cladinoside (4.59 g.) were dissolved in dry acetonitrile (15 mL) under dry argon. A solution of anhydrous silver trifluoromethanesulphonate (3.46g) in dry acetonitrile (60.1 mL) was added and the mixture was stirred under dry argon at 25°C for 21 h. The mixture was evaporated to dryness and the residue was extracted with ethyl acetate and filtered. The ethyl acetate extract was evaporated to dryness and the residue was taken up in dichloromethane and washed with water the pH being adjusted to 10. The organic layer was dried ($MgSO_4$), filtered and evaporated to dryness. The residue was chromatographed on a silica gel column (60x2.5 cm.) using 5% increasing to 20% ethyl acetate in dichloromethane (v/v) as the eluant. Overlap fractions were rechromatographed on a silica gel column (30x2.5 cm.) using 15% ethyl acetate in dichloromethane (v/v) as the eluant to give 2″,4″,4‴-tri-O-acetyl-20-deoxo-20-dihydro-O-(4′-O-phenoxyacetyl-α-L-cladinosyl)-(1→3)-desmycosin (1.26 g., 52%) as a colourless amorphous solid (Anal.: found: C, 62.43; H, 7.89; N, 1.24%; $C_{61}H_{93}NO_{21}$ requires: C, 62.28; H, 7.97; N, 1.19%); MS: m/z 1176 ($MH^+$); Rotation: $[\alpha]_D^{26}$ -33.6° ($CHCl_3$); UV: $\lambda_{max}$ ($CF_3CH_2OH$) 210 nm (ε10,676), 282 nm (ε23,146); IR: $\nu_{max}$ ($CDCl_3$) 1740, 1673, 1591, 1235, 1164, 1050, 1016 $cm^{-1}$; $^1$H-NMR: $\delta_H$ ($CDCl_3$) 0.87 (3H,t,$J_{19,20\text{-}CH_3}$ 7Hz, 20-$CH_3$), 0.89 (3H,t,$J_{16,17\text{-}CH_3}$ 7Hz, 17-$CH_3$), 0.94 (3H,d,J 7Hz, $CH_3$), 1.05 (3H,s,3′-$CH_3$), 1.12 (6H,d,J 7Hz, $CH_3$), 1.16 (3H,d,J 7Hz, $CH_3$), 1.17 (3H,d,J 7Hz, $CH_3$), 1.78 (3H,d,$J_{12\text{-}CH_3,13}$ 1Hz, 12-$CH_3$), 2.00 (3H,s,4″-$OCOCH_3$), 2.04 (3H,s,2″-$OCOCH_3$), 2.10 (3H,s,4‴-$OCOCH_3$), 2.34 (6H,s,3″-$N(CH_3)_2$), 3.18 (3H,s,3′-$OCH_3$), 3.44 (3H,S,2‴-$OCH_3$), 3.50 (3H,s,3‴-$OCH_3$), 4.42 (1H,dd,$J_{4'''ax,5'''ax}$ 10Hz, $J_{3'''ax,4'''ax}$ 2.5Hz, $H_{4'''}$ax), 4.46 (1H,d,$J_{1''ax,2''ax}$ 8Hz, $H_{1''}$ax), 4.60 (1H,d,$J_{1'''ax,2'''ax}$ 8Hz, $H_{1'''}$ax), 4.64 (1H,d,J 16Hz, 4′-$OCOCH_2OC_6H_5$), 4.70 (1H,d,J 16Hz, 4′-$OCOCH_2OC_6H_5$), 4.73 (H,dd,$J_{3''ax,4''ax}$ = $J_{4''ax,5''ax}$ = 10Hz, $H_{4''}$ax), 4.76

(1H,d,$J_{4'ax,5'ax}$ 10Hz, $H_{4'ax}$), 4.84 (1H,dd,$J_{1'eq,2'eq}$ 1Hz, $J_{1'eq,2'ax}$ 4Hz, $H_{1'eq}$), 4.86 (1H,m,$H_{15}$), 4.88 (1H,dd,$J_{1''ax,2''ax}$ 8Hz, $J_{2''ax,3''ax}$ 10Hz, $H_{2''ax}$), 5.81 (1H,dq,$J_{12\text{-}CH_3,13}$ 1Hz, $J_{13,14}$ 10Hz, $H_{13}$), 6.22 (1H,d,$J_{10,11}$ 16Hz, $H_{10}$), 6.89 (2H,d,J 9Hz, 4'-$OCOCH_2OC_6H_5$ ($H_2$ and $H_6$)), 6.97 (1H,dd,J 9Hz, J 9Hz, 4'-$OCOCH_2OC_6H_5$ ($H_4$)), 7.24 (2H,dd,J 9Hz, J 9Hz, 4'-$OCOCH_2OC_6H_5$ ($H_3$ and $H_5$)), and 7.27 (1H,d,$J_{10,11}$ 16Hz, $H_{11}$). The more polar fractions afforded unreacted 2',4',4''-tri-O-acetyl-20-deoxo-20-dihydrodesmycosin (490 mg., 27%).

EXAMPLE 8

O-α-L-Cladinosyl-(1→3)-20-deoxo-20-dihdyrodesmycosin

(i) 2'',4'',4'''-Tri-O-acetyl-(4'-O-acetyl-α-L-cladinosyl)-(1→3)-20-deoxo-20-dihydrodesmycosin (Example 7-(c)) (456 mg.) was dissolved in methanol (11.1 mL) containing 1,8-diazabicyclo[5.4.0]undec-7-ene(0.21 mL) (1.93% w/v solution) and the mixture was stirred under argon at 25°C for 45 h. The mixture was evaporated to dryness and the residue was chromatographed first on a silica gel column (30x2 cm.) using 3.3% methanol in chloroform (v/v) as the eluant, and then on a silica gel column (30x2 cm.) using 4% methanol in chloroform (v/v) as the eluant to give O-α-L-cladinosyl-(1→3)-20-deoxo-20-dihydrodesmycosin (241 mg) which contained about 33% of O-α-L-cladinosyl-(1→3)-20-deoxo-20-dihdyrodesmycosin seco acid methyl ester; MS: m/z 948 ($MH^+$) which could not be separated in any of the chromatographic systems tried. Additional signals arising from the seco acid methyl ester were also evident in the $^1H$-NMR: $\delta_H$ ($CDCl_3$) 1.88 (3H,s,$J_{12\text{-}CH_3,13}$ 1Hz, 12-$CH_3$), 3.23 (3H,s,3'-$OCH_3$), 3.55 (3H,s,1-$COOCH_3$), 4.26 (1H,d,$J_{1''ax,2''ax}$ 8Hz, $H_{1''ax}$), 4.57 (1H,d,$J_{1'''ax,2'''ax}$ 8Hz, $H_{1'''ax}$), 5.97 (1H,dq, $J_{12\text{-}CH_3 13}$ 1Hz, $J_{13,14}$ 10Hz, $H_{13}$), 6.23 (1H,d,$J_{10,11}$ 16Hz, $H_{10}$), and 7.64 (1H,d,$J_{10,11}$ 16Hz, $H_{11}$).

(ii) 2'',4'',4'''-Tri-O-acetyl-20-deoxo-20-dihydro-O-(4'-O-phenoxyacetyl-α-L-cladinosyl)-(1→3)-desmycosin (Example 7(d)) (683 mg) was dissolved in methanol (81.7 mL) containing triethylamine (1.633 mL)(2% w/v solution) and the mixture was stirred at 25°C for 94 h. The solution was evaporated to dryness and the residue was chromatographed on a silica gel column (30x2.5 cm) using 4% methanol in chloroform (v/v) as the eluant to give O-α-L-cladinosyl-(1→3)-20-deoxo-20-dihydrodesmycosin (458 mg., 86%) as a colorless amorphous solid (Found: C, 61.11; H, 8.66; N, 1.43%; $C_{47}H_{81}NO_{16}$ requires: C, 61.62; H, 8.91; N, 1.53%); MS: m/z 916 ($MH^+$); Rotation: $[\alpha]_D^{26}$ -45.0° ($CHCl_3$); UV: $\lambda_{max}$ ($CH_3OH$) 282 nm (ε19,398), IR: $\nu_{max}$ ($CDCl_3$) 3590, 3533, 1736, 1673, 1591, 1160, 1074, 1058 $cm^{-1}$; $^1H$-NMR: $\delta_H$ ($CDCl_3$): 0.89 (3H, t, $J_{19,20\text{-}CH_3}$ 7Hz, 20-$CH_3$), 0.91 (3H,t,$J_{16,17\text{-}CH_3}$ 7Hz, 17-$CH_3$), 1.02 (3H,d,J 7Hz, $CH_3$), 1.17 (3H,s,3'-$CH_3$), 1.19 (3H,d,J 7Hz, $CH_3$), 1.25 (3H,d,J 7Hz, $CH_3$), 1.26 (3H,d,J 7Hz, $CH_3$), 1.27 (3H,d,J 7Hz, $CH_3$), 1.79 (3H,d,$J_{12\text{-}CH_3,13}$ 1Hz, 12-$CH_3$), 2.55 (6H,s,3''-$N(CH_3)_2$), 3.16 (3H,s,3'-$OCH_3$), 3.46 (3H,s,2'''-$OCH_3$), 3.60 (3H,s,3'''-$OCH_3$), 4.32 (1H,d,$J_{1''ax,2''ax}$ 8Hz, $H_{1''ax}$), 4.54 (1H,d,$J_{1'''ax,2'''ax}$ 8Hz, $H_{1'''ax}$), 4.77 (1H,dd,$J_{1'eq,2'eq}$ 1Hz, $J_{1'eq,2'ax}$ 4Hz, $H_{1'eq}$), 4.88 (1H,m,$H_{15}$), 5.84 (1H,dq,$J_{12\text{-}CH_3,13}$ 1Hz, $J_{13,14}$ 10Hz, $H_{13}$), 6.21 (1H,d,$J_{10,11}$ 16Hz, $H_{10}$), and 7.27 (1H,d,$J_{10,11}$ 16Hz, $H_{11}$).

EXAMPLE 9

O-α-L-Cladinosyl-(1→3)-19-deformyldesmycosin

(a) 19-Deformyldesmycosin

Desmycosin (Merck Index, Tenth Edition p. 1405) (18.9 g) was dissolved in degassed benzene (580 mL) and tris(triphenylphosphine)rhodium chloride (29.5 g.) was added. The mixture was heated under dry nitrogen at 90°C for 16 h. The mixture was diluted with ethyl acetate and extracted twice with 0.2N hydrochloric acid. The aqueous acid layer was adjusted to pH 10 and extracted with dichloromethane. The latter was dried ($MgSO_4$), filtered and evaporated to dryness. The residue was chromatographed on a silica gel column (60x5 cm.) using 5% methanol in chloroform (v/v) as the eluant to give 19-deformyldesmycosin (13.2 g., 73%) as a colorless amorphous solid (Anal.: found: C, 57.05; H, 7.99; N, 1.62%. $C_{38}H_{65}NO_{13}$. 0.5

$CHCl_3$ requires: C, 56.79; H, 8.15; N, 1.74%); MS: m/z 744 ($MH^+$); Rotation: $[\alpha]_D^{26}$ 0° ($CHCl_3$); UV: $\lambda_{max}$ ($CH_3OH$) 282 nm (ε20,676); IR: $\nu_{max}$ ($CDCl_3$) 3540, 3450, 1705, 1670, 1590, 1185, 1163, 1055 $cm^{-1}$; $^1H$-NMR: $\delta_H$ ($CDCl_3$) 0.92 (3H,t,$J_{16,17\text{-}CH_3}$ 7Hz, 17-$CH_3$), 1.02 (3H,d,J 7Hz, $CH_3$), 1.03 (3H,d,J 7Hz, $CH_3$), 1.18 (3H,d,J 7Hz, $CH_3$), 1.26 (3H,d,J 7Hz, $CH_3$), 1.30 (3H,d,J 7Hz, $CH_3$), 1.78 (3H,d,$J_{12\text{-}CH_3,13}$ 1Hz, 12-$CH_3$), 2.53 (6H,s,3′-$N(CH_3)_2$), 3.48 (3H,s,2″-$OCH_3$), 3.60 (3H,s,3″-$OCH_3$), 4.28 (1H,d,$J_{1'ax,2'ax}$ 8Hz, $H_{1'}ax$), 4.55 (1H,d,$J_{1''ax,2''ax}$ 8Hz, $H_{1''}ax$), 4.97 (1H,m,$H_{15}$), 5.82 (1H,dq,$J_{12\text{-}CH_3,13}$ 1Hz, $J_{13,14}$ 10Hz, $H_{13}$), 6.24 (1H,d,$J_{10,11}$ 16Hz, $H_{10}$), and 7.24 (1H,d,$J_{10,11}$ 16Hz, $H_{11}$).

(b) 2′,4′-Di-O-acetyl-19-deformyldesmycosin

19-Deformyldesmycosin (12 g) was dissolved in dry acetone (100 mL) and acetic anhydride (7.6 mL) was added. The mixture was allowed to remain at 25°C for 17 h. The solution was evaporated to dryness and the residue was azeotroped with toluene to give 2′,4′-di-O-acetyl-19-deformyldesmycosin (11.5 g., 86%) as a colorless amorphous solid that was used without further purification.

An aliquot (800 mg) was chromatographed on a silica gel column (15x2.5 cm.) using 10% acetone in hexane as the eluant to give 2′,4′-di-O-acetyl-19-deformyldesmycosin (680 mg) (Anal.: found: C, 60.86, H, 8.49; N, 1.87%. $C_{42}H_{69}NO_{15}$ requires: C, 60.92; H, 8.40; N, 1.69%); MS: m/z 828 ($MH^+$); Rotation: $[\alpha]_D^{26}$ +11.9° ($CHCl_3$); UV: $\lambda_{max}$ ($CF_3CH_2OH$) 283 nm (ε23,838); IR: $\nu_{max}$ ($CDCl_3$) 3543, 1742, 1712, 1675, 1590, 1233, 1184, 1174, 1055 $cm^{-1}$; $^1H$-NMR: $\delta_H$ ($CDCl_3$) 0.92 (3H,t,$J_{16,17\text{-}CH_3}$ 7Hz, 17-$CH_3$), 0.93 (3H,d,J 7Hz, $CH_3$), 1.03 (3H,d,J 7Hz, $CH_3$), 1.14 (3H,d,J 7Hz, $CH_3$), 1.18 (3H,d,J 7Hz, $CH_3$), 1.25 (3H,d,J 7Hz, $CH_3$), 1.77 (3H,d,$J_{12\text{-}CH_3,13}$ 1Hz, 12-$CH_3$), 2.05 (6H,s,2′and 4′-$OCOCH_3$), 2.34 (6H,s,3′-$N(CH_3)_2$), 3.46 (3H,s,2″-$OCH_3$), 3.60 (3H,s,3″-$OCH_3$), 4.33 (1H,d,$J_{1'ax,2'ax}$ 8Hz, $H_{1'}ax$), 4.54 (1H,d,$J_{1''ax,2''ax}$ 8Hz, $H_{1''}ax$), 4.74 (1H,dd,$J_{3'ax,4'ax}$ = $J_{4'ax,5'ax}$ = 10Hz, $H_{4'}ax$), 4.90 (1H,dd,$J_{1'ax,2'ax}$ 8Hz, $J_{2'ax,3'ax}$ 10Hz, $H_{2'}ax$), 4.92 (1H,m,$H_{15}$), 5.83 (1H,d,$J_{10,11}$ 16Hz, $H_{10}$), and 7.27 (1H,d,$J_{10,11}$ 16Hz, $H_{11}$).

(c) 2′,4′,4″-Tri-O-acetyl-19-deformyldesmycosin

2′,4′-Di-O-acetyl-19-deformyldesmycosin (10.3 g.), 4-dimethylaminopyridine (304 mg) and triethylamine (17.3 mL) were dissolved in dry dichloromethane (300 mL) and acetic anhydride (1.174 mL) was added. The mixture was allowed to remain at 25°C for 22 h. under dry argon. The mixture was extracted with water at pH 8.5 and the dichloromethane layer was dried ($MgSO_4$), filtered and evaporated to dryness. The residue was chromatographed on a silica gel column (60x5 cm.) using 10% increasing to 15% acetone in hexane (v/v) as the eluant to give 2′,4′,4″-tri-O-acetyl-19-deformyldesmycosin (8.1 g., 75%) as a colorless amorphous solid (Anal.: found: C, 60.88; H, 8.42; N, 1.31%; $C_{44}H_{71}NO_{16}$ requires: C, 60.74; H, 8.23; N, 1.61%); MS: m/z 870 ($MH^+$); Rotation: $[\alpha]_D^{26}$ +25.3° ($CHCl_3$); UV: $\lambda_{max}$ ($CF_3CH_2OH$) 284 nm (ε22,479); IR: $\nu_{max}$ ($CDCl_3$) 3520, 1737, 1672, 1590, 1234, 1181, 1161, 1050 $cm^{-1}$; $^1H$-NMR: $\delta_H$ ($CDCl_3$) 0.90 (3H,t,d,$J_{16,17\text{-}CH_3}$ 7Hz, 17-$CH_3$), 0.94 (3H,d,J 7Hz, $CH_3$), 1.03 (3H,d,J 7Hz, $CH_3$), 1.13 (3H,d,J 7Hz, $CH_3$), 1.16 (3H,d,J 7Hz, $CH_3$), 1.18 (3H,d,J 7Hz, $CH_3$), 1.77 (3H,d,$J_{12\text{-}CH_3,13}$ 1Hz, 12-$CH_3$), 2.05 (6H,s,2′ and 4″-$OCOCH_3$), 2.10 (3H,s,4″-$OCOCH_3$), 2.33 (6H,s,3′-$N(CH_3)_2$), 3.46 (3H,s,2″-$OCH_3$), 3.51 (3H,s,3″-$OCH_3$), 4.33 (1H,d,$J_{1'ax,2'ax}$ 8Hz, $H_{1'}ax$), 4.42 (1H,dd,$J_{3''eq,4''ax}$ 3Hz, $J_{4''ax,5''ax}$ 10Hz, $H_{4''}ax$), 4.60 (1H,d,$J_{1''ax,2''ax}$ 8Hz, $H_{1''}ax$), 4.75 (1H,dd,$J_{3'ax,4'ax}$ = $J_{4'ax,5'ax}$ = 10Hz, $H_{4'}ax$), 4.88 (1H,dd,$J_{1'ax2'ax}$ 8Hz, $J_{2'ax,3'ax}$ 10Hz, $H_{2'}ax$), 4.92 (1H,m,$H_{15}$), 5.82 (1H,dq,$J_{12\text{-}CH_3,13}$ 1Hz, $J_{13,14}$ 10Hz, $H_{13}$), 6.28 (1H,d,$J_{10,11}$ 16Hz, $H_{10}$) and 7.26 (1H,d,$J_{10,11}$ 16Hz, $H_{11}$). The more polar fractions afforded unreacted 2′,4′-di-O-acetyl-19-deformyldesmycosin (1.35 g., 13%).

(d) 2″,4″,4‴-Tri-O-acetyl-19-deformyl-O-(4′-O-phenoxyacetyl-α-L-cladinosyl)-(1→3)-desmycosin

2′,4′,4″-Tri-O-acetyl-19-deformyldesmycosin (2.5 g) and 1-deoxy-4-O-phenoxyacetyl-1-(pyridyl-2-thio)-α- and -β-L-cladinoside (6.38 g.) were dissolved in dry acetonitrile (50 mL) under dry argon. A solution of anhydrous silver trifluoromethanesulphonate (4.8 g.) dissolved in dry acetonitrile (50 mL) was added and the mixture was stirred under dry argon at 25°C for 20 h. The mixture was evaporated to dryness and the residue was extracted with ethyl acetate and filtered. The ethyl acetate was removed in vacuo and the

residue was taken up in dichloromethane and washed with water, the pH being adjusted to 10. The organic layer was dried ($MgSO_4$), filtered and evaporated to dryness. The residue was chromatographed on a silica gel column (30x2.5 cm) using 5% increasing to 20% ethyl acetate in dichloromethane (v/v) as the eluant. The product was rechromatographed on a silica gel column (30x2.5 cm.) using 20% ethyl acetate in dichloromethane as the eluant to give 2″,4″,4‴-tri-O-acetyl-19-deformyl-O-(4′-O-phenoxyacetyl-α-L-cladinosyl)-(1→3)-desmycosin (2.04 g., 61%) as a colorless amorphous solid (Anal.: found: C, 62.00; H, 7.84; N, 1.22%; $C_{60}H_{91}NO_{21}$ requires: C, 62.00; H, 7.89; N, 1.21%); MS: m/z 1162 ($MH^+$); Rotation: $[\alpha]_D^{26}$ -23.1° ($CHCl_3$); UV: $\lambda_{max}$ ($CF_3CH_2OH$) 210 nm (ε7,561), 283nm (ε17,856); IR: $\nu_{max}$ ($CDCl_3$) 1737, 1673, 1590, 1234, 1188, 1160, 1050, 1012 $cm^{-1}$; $^1$H-NMR: $\delta_H$ ($CDCl_3$) 0.90 (3H,t,$J_{16,17\text{-}CH_3}$ 7Hz, 17-$CH_3$), 0.92 (3H,d,J 7Hz, $CH_3$), 1.03 (3H,d,J 7Hz, $CH_3$), 1.05 (3H,s,3′-$CH_3$), 1.11 (3H,d,J 7Hz, $CH_3$), 1.14 (3H,d,J 7Hz, $CH_3$), 1.17 (3H,d,J 7Hz, $CH_3$), 1.18 (3H,d,J 7Hz, $CH_3$), 1.76 (3H,d,$J_{12\text{-}CH_3,13}$ 1Hz, 12-$CH_3$), 2.02 (3H,s,4″-$OCOCH_3$), 2.04 (3H,s,2″-$OCOCH_3$), 2.12 (3H,s,4‴-$OCOCH_3$), 2.35 (6H,s,3″-$N(CH_3)_2$), 3.21 (3H,s,3′-$OCH_3$), 3.44 (3H,s,2‴-$OCH_3$), 3.51 (3H,s,3‴-$OCH_3$), 4.37 (1H,d,$J_{1''ax,2''ax}$ 8Hz, $H_{1''ax}$), 4.41 (1H,dd,$J_{3'''eq,4'''ax}$ 2.5Hz, $J_{4'''ax,5'''ax}$ 10Hz, $H_{4'''ax}$), 4.60 (1H,d,$J_{1'''ax,2'''ax}$ 8Hz, $H_{1'''ax}$), 4.66 (1H,d,J 16Hz, 4′-$OCOCH_2OC_6H_5$), 4.71 (1H,d,J 16Hz, 4′-$OCOCH_2OC_6H_5$), 4.75 (1H,dd,$J_{3''ax,4''ax}$ = $J_{4''ax,5''ax}$ = 10Hz, $H_{4''ax}$), 4.76 (1H,d,$J_{4'ax,5'ax}$ 10Hz, $H_{4'ax}$), 4.87 (1H,m,$H_{15}$), 4.90 (1H,dd,$J_{1''ax,2''ax}$ 8Hz, $J_{2''ax,3''ax}$ 10Hz, $H_{3''ax}$), 4.97 (1H,dd,$J_{1'eq,2'eq}$ 1Hz, $J_{1'eq,2'ax}$ 4Hz, $H_{1'eq}$), 5.80 (1H,dq,$J_{12\text{-}CH_3,13}$ 1Hz, $J_{13,14}$ 10Hz, $H_{13}$), 6.24 (1H,d,$J_{10,11}$ 16Hz, $H_{10}$), 6.90 (2H,d,J 9Hz,4′-$OCOCH_2OC_6H_5$ ($H_2$ and $H_6$)), 6.97 (1H,dd,J 9Hz, J 9Hz, 4′-$OCOCH_2OC_6H_5$ ($H_4$)), 7.27 (2H,dd,J 9Hz, J 9Hz 4′-$OCOCH_2OC_6H_5$ ($H_3$ and $H_5$)), and 7.28 (1H,d,$J_{10,11}$ 16Hz, $H_{11}$). The more polar fractions afforded unreacted 2′,4′,4″-tri-O-acetyl-19-deformyldesmycosin (450 mg., 18%).

(e) O-α-L-Cladinosyl-(1→3)-19-deformyldesmycosin

2″,4″,4‴-Tri-O-acetyl-19-deformyl-O-(4′-O-phenoxyacetyl-α-L-cladinosyl)-(1→3)-desmycosin (1.9g) was dissolved in methanol (165 mL) containing triethylamine (4.546 mL) (2% w/v solution) and the mixture was allowed to remain at 25°C for 72 h. The solution was evaporated to dryness and the residue was taken up in dichloromethane and washed with water, the pH being adjusted to 10. The organic layer was dried ($MgSO_4$), filtered and evaporated to dryness. The residue was chromatographed on a silica gel column (60x2.5 cm.) using 4% methanol in chloroform (v/v) as the eluant. The resulting product was rechromatographed on a silica gel column (15x2.5 cm.) using ethyl acetate as the eluant to afford O-α-L-cladinosyl-(1→3)-19-deformyldesmycosin (1.28g., 87%) as a colourless amorphous solid (Anal.: found: C, 61.40; H, 8.99; N, 1.45%; $C_{46}H_{79}NO_{16}$ requires: C, 61.24; H, 8.83; N, 1.55%); MS: m/z 902 ($MH^+$); Rotation: $[\alpha]_D^{26}$ -35.6° ($CHCl_3$); UV: $\lambda_{max}$ ($CH_3OH$) 382nm (ε20,942); IR: $\nu_{max}$ ($CDCl_3$) 3600, 3558, 3450, 1738, 1732, 1674, 1592, 1160, 1058, 1010 $cm^{-1}$; $^1$H-NMR: $\delta_H$ ($CDCl_3$) 0.92 (3H,t,$J_{16,17\text{-}CH_3}$ 7Hz, 17-$CH_3$), 1.02 (3H,d,J 7Hz, $CH_3$), 1.07 (3H,d,J 7Hz, $CH_3$), 1.17 (3H,s,3′-$CH_3$), 1.19 (3H,d,J 7Hz, $CH_3$), 1.26 (3H,d,J 7Hz, $CH_3$), 1.27 (3H,d,J 7Hz, $CH_3$), 1.29 (3H,d,J 7Hz, $CH_3$), 1.77 (3H,d,$J_{12\text{-}CH_3,13}$ 1Hz, 12-$CH_3$), 2.50 (6H,s,3″-$N(CH_3)_2$), 3.17 (3H,s,3′-$OCH_3$), 3.45 (3H,s,2‴-$OCH_3$), 3.60 (3H,s,3‴-$OCH_3$), 4.24 (1H,d,$J_{1''ax,2''ax}$ 8Hz, $H_{1''ax}$), 4.53 (1H,d,$J_{1'''ax,2'''ax}$ 8Hz, $H_{1'''ax}$), 4.86 (1H,m,$H_{15}$), 4.90 (1H,dd,$J_{1'eq,2'eq}$ 1Hz, $J_{1'eq,2'ax}$ 4Hz, $H_{1'eq}$), 5.82 (1H,dq,$J_{12\text{-}CH_3,13}$ 1Hz, $J_{13,14}$ 10Hz, $H_{13}$), 6.23 (1H,d,$J_{10,11}$ 16Hz, $H_{10}$), and 7.26 (1H,d,$J_{10,11}$ 16Hz, $H_{11}$).

EXAMPLE 10

α-and -β-O-Tetrahydropyranyl-(1→3)-rosaramicin

(a) (1R/S)-S-(Pyridyl-2-thio)-tetrahydropyran

Dihydropyran (dry, redistilled) (48 mL) and 2-mercaptopyridine (6.66g.) were dissolved in dry dichloramethane (70 mL) under dry nitrogen. Biorad AG50W-X8 (200-400 mesh) sulphonic acid cation exchange resin (H+ form) (23 g.)(dried by azeotroping with toluene) was added and the mixture was stirred in the dark at 25°C for 72 h. The resin was filtered off, washed with dichloromethane and the combined filtrates were evaporated to dryness. The residue was chromatographed on silica gel column (280 g.) using initially 50% hexane in dichloromethane, followed by 25% hexane in dichloromethane and finally 1% ethyl acetate

in dichloromethane (all v/v) as the eluant to give a 30:70 mixture (w/w) of (1R/S)-N-(pyridyl-2-thione)-tetrahydropyran and (1R/S)-S-(pyridyl-2-thio)-tetrahydropyran (8.31g.) The more polar fractions afforded additional pure (1R/S)-S-(pyridyl-2-thio)-tetrahydropyran (2.4 g.) upon rechromatography as described above. In order to obtain analytically pure samples of each component of the mixture, a portion of the mixture (0.65 g.) was rechromatographed on an alumina column (100 mL capacity) using 4% increasing to 6% and then 8% of ethyl acetate in hexane (all v/v) as the eluant to give in the order of elution (1R/S)-S-(pyridyl-2-thio)-tetrahydropyran (177 mg.) as a yellow oil; MS: m/z 196 ($MH^+$); Rotation: $[\alpha]_D^{26}$ 0° ($CH_3OH$); UV: $\lambda_{max}$ ($CH_3OH$) 242 nm (ε9,055) 285 nm (ε4,348); IR: $\nu_{max}$ ($CHCl_3$), 1573, 1555, 1427, 1416, 1185, 1121, 1100, 1076, 1038, 1008 $cm^{-1}$; $^1$H-NMR: $\delta_H$ ($CDCl_3$) 1.60-2.00, 2.12 (5H and 1H, m, $H_2$, $H_3$, $H_4$), 3.71 (1H,ddd,$J_{5ax,5eq}$, 12Hz, $J_{4eq,5ax}$ 4Hz, $J_{4ax,5ax}$ 10.5Hz, $H_{5ax}$), 4.14 (1H,ddd,$J_{5ax,5eq}$ 12Hz, $J_{4eq,5eq} = J_{4ax,5eq} = 6$Hz, $H_{5eq}$), 5.99 (1H,dd,$J_{1eq,2eq}$ 5Hz, $J_{1eq,2ax}$ 5.5Hz, $H_{1eq}$), 7.06 (1H,ddd,$J_{4,5}$ 8Hz, $J_{5,6}$ 5Hz, $J_{3,5}$ 1.2Hz, 1-S-$C_5H_4$N-($H_5$)), 7.34 (1H,ddd,$J_{3,4}$ 8Hz, $J_{3,5} = J_{3,6} = 1.2$Hz, 1-S-$C_5H_4$N($H_3$)), 7.54 (1H,ddd,$J_{3,4} = J_{4,5} = 8$Hz, $J_{4,6}$2Hz, 1-S-$C_5H_4$N($H_4$)), and 8.51 (1H,ddd,$J_{5,6}$ 5Hz, $J_{3,6}$ 1.2Hz, $J_{4,6}$ 2Hz, 1-S-$C_5H_4$N($H_6$)), and (1R/S)-N-(pyridyl-2-thione)-tetrahydropyran (233 mg.) as a yellow oil; MS: m/z 196 ($MH^+$); Rotation: $[\alpha]_D^{26}$ -0.6° ($CH_3OH$); UV: $\lambda_{max}$ ($CH_3OH$) 283 nm (ε11,029), 360 nm (ε6,290); IR: $\nu_{max}$ ($CHCl_3$) 1614, 1527, 1452, 1419, 1253, 1207, 1180, 1115, 1079, 1042 $cm^{-1}$; $^1$H-NMR: $\delta_H$ ($CDCl_3$) 1.24, 1.60-2.08, 2.50 (1H, 4H and 1H, m, $H_2$, $H_3$, $H_4$), 3.80 (1H,ddd,$J_{5ax,4eq}$ 4.5Hz, $J_{5ax,4ax} = J_{5ax,5eq} = 11$Hz, $H_{5ax}$), 4.24 (1H,ddd,$J_{5eq,5ax}$ 11Hz, $J_{5eq,4eq} = J_{5eq,4ax} = 2$Hz, $H_{5eq}$), 6.70 (1H,dd,$J_{1ax,2ax}$ 10.5Hz, $J_{1ax,2eq}$ 2.5Hz, $H_{1ax}$), 6.73 (1H,ddd,$J_{4,5} = J_{5,6} = 7$Hz, $J_{3,5}$ 1Hz, 1-S-$C_5H_4$NS-($H_5$)), 7.19 (1H,ddd,$J_{3,4}$ 9Hz, $J_{4,5}$ 7Hz, $J_{4,6}$ 2Hz, 1-S-$C_5H_4$N($H_4$)), 7.68 (1H,ddd,$J_{3,4}$ 9Hz, $J_{3,5} = J_{3,6} = 1$Hz, 1-S-$C_5H_4$N($H_3$)) and 8.04 (1H,ddd,$J_{5,6}$ 7Hz, $J_{4,6}$ 2Hz, $J_{3,6}$ 1Hz, 1-S-$C_5H_4$N($H_6$)).

(b) α-and β-O-Tetrahydropyranyl-(1→3)-rosaramicin

2′-O-Acetylrosaramicin (prepared by acetylation of rosaramicin using acetic anhydride in acetone by the method described in H. Reimamm, et al. J. Chem. Soc., Chem. Comm., 1972, 1270) (2g.), anhydrous silver trifluoromethanesulphonate (6 g.) and dry triethylamine (2.8 mL) were dissolved in dry acetonitrile (30 mL). (1R/S)-S-(pyridyl-2-thio)-tetrahydropyran (4 ML) was added and the mixture was stirred under dry nitrogen at 25°C for 24 h. The reaction mixture was evaporated to dryness and the residue was taken up in dichloromethane and washed with 5% aqueous sodium bicarbonate. The dichloromethane layer was filtered, washed with water, dried ($Na_2SO_4$), filtered and evaporated to dryness. The residue was triturated with ethyl acetate and filtered. The solids were washed with ethyl acetate and the combined filtrates were evaporated to dryness. The residue was chromatographed on a silica gel column (200 g.) using 10%, 20%, 25% and finally 45% acetone in toluene (v/v) as the eluant to give a ca. 8:5 mixture of the less polar and more polar diastereoisomers of 2′-O-acetyl-O-tetrahydropyranyl-(1→3)-rosaramicin (1.16 g., 51%). The early fractions that were rich in the less polar diastereoisomer (327 mg) were rechromatographed on a silica gel column (32 g.) using acetone: dichloromethane: toluene: (v/v/v) (20:60:44) as the eluant to give the less polar diastereoisomer (190 mg). The latter was taken up in methanol (1.5 mL) and allowed to remain at 25°C for 17 h. The mixture was evaporated to dryness to give O-tetrahydropyranyl-(1→3)-rosaramicin as a colourless amorphous solid (Found: C, 64.56; H, 8.78; N, 2.27. $C_{36}H_{59}NO_{10}$ requires: C, 64.94; H, 8.93; N, 2.10%; MS: m/z 666 ($MH^+$); Rotation: $[\alpha]_D^{26}$ -42.5° ($CHCl_3$); UV: $\lambda_{max}$ ($CH_3OH$) 240 nm (ε12,271); IR: $\nu_{max}$ ($CDCl_3$) 3440, 1740, 1720, 1687, 1619, 1167, 1118, 1076, 1032, 986 $cm^{-1}$; $^1$H-NMR: $\delta_H$ ($CDCl_3$) 0.89 (3H,t,$J_{16,17}$-$CH_3$ 7Hz, 17-$CH_3$), 1.11 (3H,d,J 7Hz, $CH_3$), 1.12 (3H,d,J 7Hz, $CH_3$), 1.13 (3H,d,J 7Hz, $CH_3$), 1.19 (3H,d,J 7Hz, $CH_3$), 1.37 (3H,s,12-$CH_3$), 2.27 (6H,s,3″-N($CH_3$)$_2$), 4.14 (1H,d,$J_{1''ax,2''ax}$ 8Hz, $H_{1''ax}$), 4.73 (1H,m,$H_{15}$), 4.76 (1H,dd,$J_{1'eq,2'eq}$ 2Hz, $J_{1'eq,2'ax}$ 7Hz, $H_{1'eq}$), 6.36 (1H,d,$J_{10,11}$ 16Hz, $H_{10}$), 6.52 (1H,d,$J_{10,11}$ 16Hz, $H_{11}$), and 9.66 (1H,s,$H_{20}$). The later fractions from the original column that were rich in the more polar diastereoisomer (327 mg) were rechromatographed on a silica gel column (40g.) using first 10%, then 14% and finally 20% acetone in dichloromethane:toluene::60:44 (v/v) as the eluant to give the more polar diastereoisomer (40 mg.). The latter was taken up in methanol (0.5 mL) and allowed to remain at 25°C for 17 h. The solution was evaporated to dryness to give O-tetrahydropyranyl-(1→3)-rosaramicin as a colourless amorphous solid; MS: m/z 666 ($MH^+$); Rotation: $[\alpha]_D^{26}$ -35.1° ($CHCl_3$); UV: $\lambda_{max}$ ($CH_3OH$) 239 nm (ε11,264); IR: $\nu_{max}$ ($CHCl_3$) 3430, 1738, 1725, 1687, 1619, 1178, 1168, 1117, 1078, 1032, 987 $cm^{-1}$; $^1$H-NMR: $\delta_H$ ($CDCl_3$) 0.90 (3H,t,$J_{16,17-CH_3}$ 7Hz, 17-$CH_3$), 1.12 (3H,d,J 7Hz, $CH_3$), 1.16 (3H,d,J 7Hz, $CH_3$), 1.18 (3H,d,J 7Hz, $CH_3$), 1.20 (3H,d,J 7Hz, $CH_3$), 1.43 (3H,s,12-$CH_3$), 2.29 (6H,s,3″-N($CH_3$)$_2$), 4.30 (1H,d,$J_{1''ax,2''ax}$ 7.5Hz, $H_{1''ax}$), 4.72 (1H,dd,$J_{1'eq,2'eq}$ 2Hz, $J_{1'eq,2'ax}$ 7.5Hz, $H_{1'eq}$), 4.84 (1H,m,$H_{15}$), 6.42 (1H,d,$J_{10,11}$ 16Hz, $H_{10}$), 6.59 (1H,d,$J_{10,11}$ 16Hz, $H_{11}$), and 9.77 (1H,s,$H_{20}$).

EXAMPLE 11 (starting materials)

3,4-Di-O-(para-methoxybenzyl)-1-deoxy-1-(pyridyl-2-thio)-α-and -β-L-digitoxoside

Follow the procedure of Brimacombe et al., J. Chem. Soc., Perkin I, 1982, 2583, to prepare methyl 3-oxo-3-dehydro-L-digitoxoside which is treated with $NaBH_4$ in methanol to prepare methyl α-L-digitoxoside. React methyl α-L-digitoxoside with more than two equivalents of para-methoxybenzyl triflate in the presence of excess 2,4,6-trimethylpyridine to obtain methyl 3,4-di-O-(para-methoxybenzyl)-α-L-digitoxoside. Contact methyl 3,4,-di-O-para-methoxybenzyl)-α-L-digitoxoside with 1N aqueous HCl at 25°C to produce 3,4-di-O-(para-methoxybenzyl)-α and β-L-digitoxose. React a solution of the 3,4,-di-O-(para-methoxybenzyl) α-and β-L-digitoxose in dichloromethane with a solution 2,2′-dipyridyldisulfide and tri-n-butyl phosphine in dichloromethane to produce the title compound.

EXAMPLE 12 (starting materials)

3-O-Acetyl-4-O-methyl-1-deoxy-1-(pyridyl-2-thio)-α-and β-L-digitoxoside

Treat methyl 3-oxo-3-dehydro-L-digitoxoside with methyltriflate in 2,4,6-trimethylpyridine to obtain methyl 4-O-para-methoxybenzyl-3-oxo-3-dehydro-L-digitoxoside. React methyl 4-O-para-methoxybenzyl-3-oxo-3-dehydro-L-digitoxoside with sodium borohydride in methanol to obtain methyl 4-O-methyl-α-L-digitoxoside. Treat methyl 4-O-methyl-α-L-digitoxoside with 0.1N aqueous HCl to obtain 4-O-methyl-α-and β-L-digitoxose which is reacted sequentially with acetic anhydride in the presence of pyridine and thereafter with 1N aqueous HCl and therafter with a solution of 2,2′-dipyridyldisulfide and tri-n-butylphosphine in dichlormethane to produce the title compound.

EXAMPLE 13 (starting materials)

3-O-Methyl-4-O-para-methoxybenzyl-1-deoxy-1-(pyridyl-2-thio)-α-and β-L-digitoxside

Treat methyl 3-oxo-3-dehydro-L-digitoxoside with para-methoxybenzyl triflate in the presence of 2,4,6-trimethylpyridine to produce methyl 4-O-para-methoxybenzyl-3-oxo-3-dehydro-L-digitoxoside which is thereafter reduced with sodium borohydride in methanol to give methyl 4-O-para-methoxybenzyl-α-L-digitoxoside. Contact methyl 4-O-para-methoxybenzyl-α-L-digitoxoside sequentially with silver (II) oxide and methyl iodide in dimethylformamide, then with 1N aqueous HCl and finally with 2,2′-dipyridyldisulfide in the presence of tri-n-butylphosphine to give the title compound.

EXAMPLE 14 (starting materials)

3,4-Di-O-Methyl-1-deoxy-1-(pyridyl-2-thio)-α-and -β-L-digitoxoside

Treat methyl α-L-digitoxoside with two equivalents of methyl triflate in the presence of 2,6-di-tert-butylpyridine to give methyl 3,4-di-O-methyl-α-L-digitoxoside which is thereafter sequentially treated with 1N aqueous HCl and then 2,2′-dipyridyldisulfide in the presence of tri-n-butylphosphine to give the title compound.

EXAMPLE 15

O-α-L-Cladinosyl-(1→3)-23-O-demycinosyl-4‴-O-iso-valeryltylosin

Treat 23,2′-di-O-acetyl-23-O-demycinosyl-4″-O-iso-valeryltylosin (prepared as described in International Publication No. WO 87/03880, published 2 July 1987) with 1-deoxy-4-O-phenoxyacetyl-1-(pyridyl-2-thio)-α- and -β-L-cladinoside (Example 5(c)) in the presence of anhydrous silver trifluoromethanesulphonate to afford 23,2″-di-O-acetyl-23-O-demycinosyl-4‴-O-iso-valeryl-O-(4′-O-phenoxyacetyl-α-L-cladinosyl)-(1→3)-tylosin. Treatment of the latter with 2% (v/v) triethylamine in methanol affords the title compound.

EXAMPLE 16

23,2″-Di-O-acetyl-O-α-L-cladinosyl-(1→3)-23-O-demycinosyl-4″-O-iso-valeryltylosin

Treat 23,2′-di-O-acetyl-23-O-demycinosyl-4″-O-iso-valeryltylosin (prepared as described in International Publication No. WO 87/03880, published 2 July 1987, especially the acylation Schemes I and II) with 1-deoxy-4-O-phenoxyacetyl-1-(pyridyl-2-thio)-α-and -β-L-cladinoside (Example 5(c)) in the presence of anhydrous silver trifluoromethanesulphonate to give 23,2‴-di-O-acetyl-23-O-demycinosyl-4″-O-iso-valeryl-O-(4′-O-phenoxyacetyl-α-L-cladinosyl)-(1→3)-tylosin. Treatment of the latter with 2% (v/v) triethylamine in methanol, followed by subsequent treatment of product with acetic anhydride in acetone, gives the title compound.

EXAMPLE 17

O-(4′-O-Acetyl-α-L-cladinosyl)-(1→3)-23-O-demycinosyltylosin

Treat 23,2′,4″-tri-O-phenoxyacetyl-23-O-demycinosyltylosin (prepared by the methods described in International Publication No. WO 87/03880, published 2 July 1987 especially the acylation Schemes I and III) with 1-deoxy-4-O-acetyl-1-(pyridyl-2-thio)-α-and -β-L-cladinoside (Example 1(d)) in the presence of anhydrous silver trifluoromethanesulphonate and subsequently with 2% (v/v) triethylamino in methanol to give the title compound.

In a similar fashion the corresponding 4′-O-propionyl, 4′-O-butyryl and O-(4′-O-iso-valeryl-α-L-cladinosyl)-(1→3)-23-O-demycinosyltylosins may be prepared by substitution of an equivalent quantity of propionic anhydride, butyric anhydride or iso-valeric anhydride for acetic anhyride in the preparation of the cladinose derivative described in Example 1 above.

EXAMPLE 18

3-O-(2-Methoxy-1-butyloxymethyl)-12,13-dehydro-12,13-deoxorosaramicin

2-Methoxy-1-butanol (prepared by standard methods known in the art, for example those described in CA, 1984, Vol. 101, 6616f and CA, 1984, Vol. 100, 209159 m.) is reacted with formaldehyde and 90% HCl to give 2-methoxy-1-butyloxymethyl chloride. The latter reacts with 2′-O-acetyl-12,13-dehydro-12,13-deoxorosaramicin of Example 2(a) in the presence of a tertiary amino base, followed by reaction with a lower alkanol such as methanol, to give the title compound.

EXAMPLE 19

O-(1-Ethoxyethyl)-(1→3)-rosaramicin

(α) 2-S-(1-Ethoxyethyl)mercaptopyridine

2-Mercaptopyridine (0.666 g) was dissolved in dry dichloromethane (7 ml.) and the solution was cooled to 0°C. Ethyl vinyl ether (5 ml) (which had been distilled over sodium) and BioRad 50Wx4 sulphonic acid resin ($H^+$ cycle) (2.3g) were added and the mixture was allowed to warm gradually to 25°C overnight. After 17h the resin was filtered off and washed with dry dichloromethane. The combined filtrates were evaporated to dryness and the residue was chromatographed on a silica gel column (50 g) using first hexane, then 50% hexane in dichloromethane (v/v), then 25% hexane in dichloromethane (v/v), then dichloromethane, then 1% ethyl acetate in dichloromethane (v/v) and finally 2% ethyl acetate in dichloromethane (v/v) to give 2-S-(1-ethoxyethyl)-mercaptopyridine, MS: m/z 184 ($MH^+$) ; $^1$H-NMR: $\delta_H$ ($CDCl_3$) 1.21 (3H,t,J 7Hz, $OCH_2CH_3$), 1.55

S

(3H),d,J 7Hz, CH-$CH_3$),

O

3.50 (2H,m, $OCH_2CH_3$), 6.78 (1H,m -1-S-$C_5H_4N$), 7.20 (1H,m,1-S-$C_5H_4N$), 7.68 (1H,m,1-S-$C_5H_4N$) and 7.95 (1H,m, 1-S-$C_5H_4N$).

(b) O-(1-Ethoxyethyl)-(1→3)-rosaramicin

2′-O-Acetylrosaramicin (100 mg), 2-S-(1-ethoxyethyl)mercaptopyridine (0.18 ml), anhydrous silver trifluoromethanesulphonate (300 mg) and dry triethylamine (0.14 ml) were dissolved in dry acetonitrile (0.18 ml) and the mixture was stirred at 25°C for 17h. The solution was evaporated to dryness and the residue was partitioned between 5% aqueous sodium bicarbonate and dichloromethane. The dichloromethane layer was washed with water, dried ($Na_2SO_4$), filtered and evaporated to dryness. The residue was chromatographed by preparative thin layer chromatography on 250 μ silica gel plates (sisc) using 45% acetone in toluene as the eluant to give 2′-O-acetyl-O-(1-ethoxyethyl)-(1→3)-rosaramicin (18 mg), MS; m/z 696 ($MH^+$). Treatment of the latter with methanol (5 ml) at 25°C for 17h. afforded O-(1-ethoxyethyl)-(1→3)-rosaramicin.

EXAMPLE 20

O-β-D-Glucopyranonyl-(1→3)-12,13-dehydro-12,13-deoxorosaramicin

Treat 2′-O-acetyl-12,13-dehydro-12,13-deoxorosaramicin with 2,3,4,6-tetra-O-acetyl-1-deoxy-1-(pyridyl-2-thio)-α-and -β-D-glucopyranoside (prepared as described in Example 1(d) starting from 2,3,4,6-tetra-O-acetyl-α-and -β-Dglucopyranose, which is prepared as described by J. Fiandor et. al., Synthesis, 1985, pp 1121-1123) in the presence of anhydrous, silver trifluoromethanesulphonate to give the β-D-glycoside, which is then deprotected as described in Example 8 to give the title compound.

## Claims

1. A compound represented by the formula I:

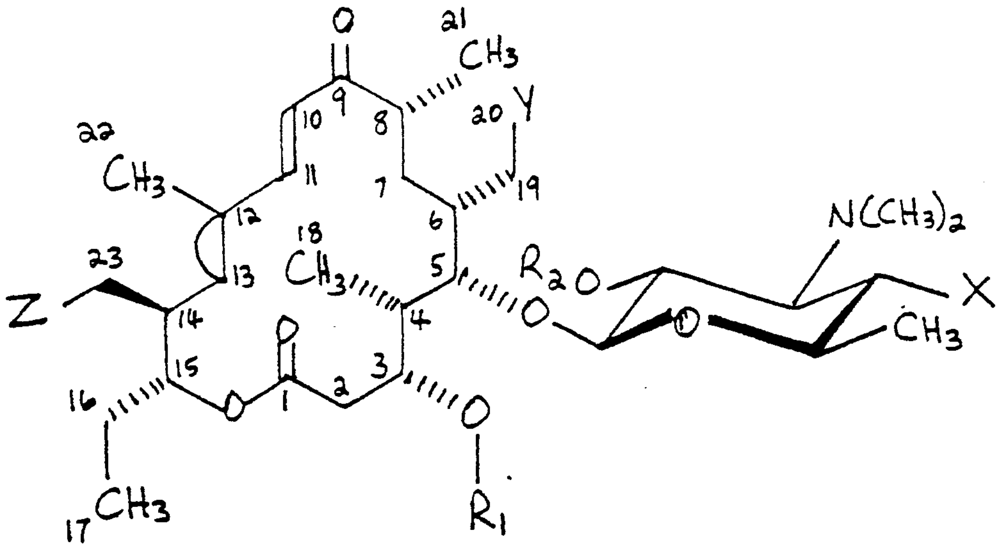

wherein $R_1$ is a glycosyl group represented by the formula II:

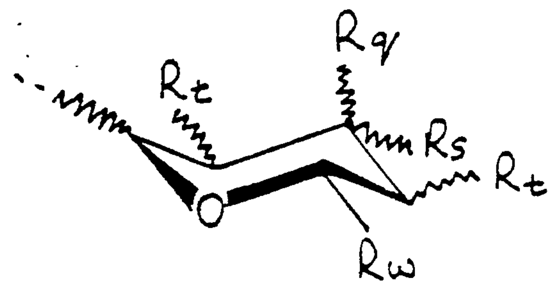

wherein $R_q$ and each $R_t$ are independently hydrogen, hydroxy, acyloxy, loweralkoxy, aralkoxy, N-loweralkylamino-carbonyloxy, N-aralkylamino carbonyloxy, or arylsulfonyloxy ($-OSO_2Ar$) and $R_s$ and $R_w$ are independently hydrogen, lower alkyl or $-CH_2OH$;
or represented by the formula IIA:

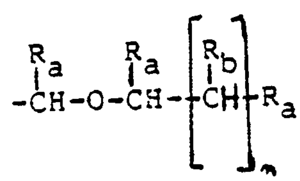

IIa

wherein each $R_a$ is independently hydrogen, lower alkyl, or $CH_2OH$ and each $R_b$ is independently hydrogen, hydroxy, acyloxy, loweralkoxy, aralkoxy, N-loweralkylaminocarbonyloxy, N-aralkylaminocarbonyloxy or arylsulfonyloxy and n is 1, 2, 3 or 4;

$R_2$ is hydrogen or an acyl group;

X is hydrogen, hydroxyl, an acyloxy group

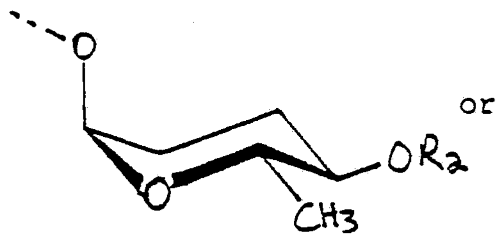 or 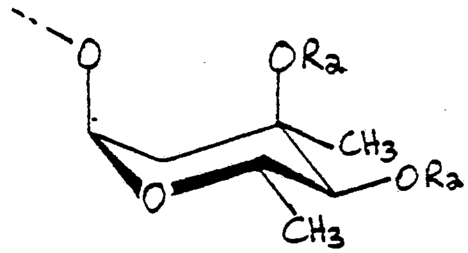

wherein each $R_2$ is independently as defined hereinabove;

Y is hydrogen, a formyl group, methyl,

HC=NNH-aralkyl, 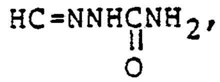,

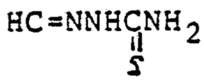 or 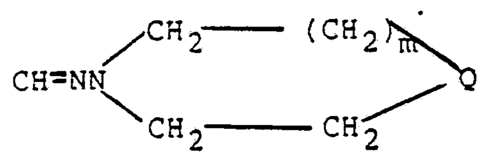

wherein m is 0,1, or 2 and Q is independently $CR_3R_4$, $NR_3$, O, S, $SO_2$, $CR_3OR_4$,

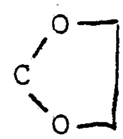

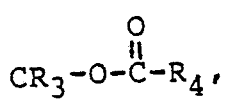, 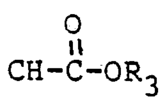, or 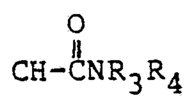

wherein $R_3$ and $R_4$ are independently hydrogen, lower alkyl, aralkyl, G-substituted aralkyl, aryl and G-substituted aryl wherein G is independently one or more of halogen, trifluoromethyl, lower alkoxy or ($C_2$-$C_7$) alkanoyl;

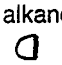 is a 12,13 double bond or a 12,13-oxo moiety;

Z is hydrogen, hydroxy, an acyloxy group, a N-N-di(loweralkyl) amino group,

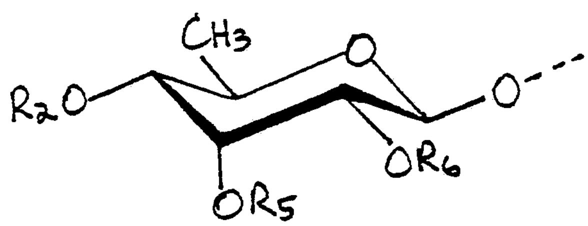,

loweralkoxycarbonyloxy, aralkoxycarbonyloxy, N-lower-alkylaminocarbonyloxy, and N-aralkylaminocarbonyloxy wherein $R_2$ is defined above and $R_5$ and $R_6$ are independently hydrogen, lower alkyl or acyl groups;

or a pharmaceutically acceptable acid addition salt thereof.

2. A compound of claim 1 wherein $R_1$ is an α-L-glycosyl group represented by formula II

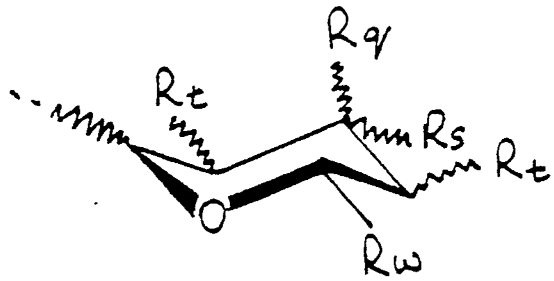

II

wherein $R_q$, $R_s$, $R_t$, and $R_w$ are as defined in claim 1.

3. A compound of claim 1 wherein $R_1$ is α-L-cladinosyl or tetrahydropyranyl,

X is hydrogen, hydroxy, or

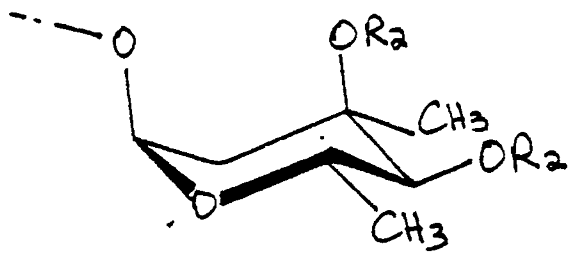

wherein $R_2$ is as defined in claim 2,

Y is methyl, or formyl,

Z is hydrogen, acyloxy, hydroxy, or

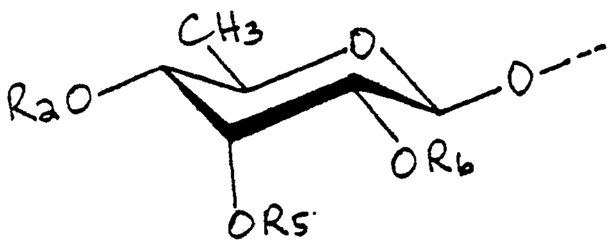

wherein $R_2$, $R_5$, and $R_6$ ar as defined in claim 1.

4. A compound of claim 1 which is O-(α-L-cladinosyl)-(1→3)-12,13-dehydro-20-dihydro-12,13,20-dideoxorosaramicin,

O-(α-L-cladinosyl)-(1→3)-12,13-dehydro-12,13-deoxorosaramicin,

O-(4′-O-acetyl-α-L-cladinosyl)-(1→3)-2″-O-acetyl-12,13-dehydro-20-dihydro-12,13,20-dideoxorosaramicin,

O-(4′-O-acetyl-α-L-cladinosyl)-(1→3)-2″-O-acetyl-12,13-dehydro-12,13-deoxorosaramicin, or

O-tetrahydropyranyl-(1→3)-rosaramicin.

5. A compound of claim 1 wherein $R_1$ is

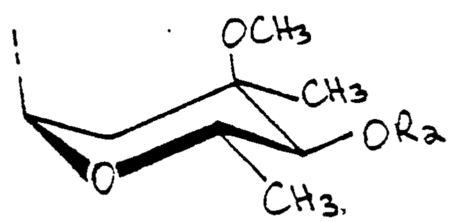

wherein $R_2$ is as defined in claim 1, X is hydroxyl or an acyloxy group, 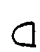 is a 12,13 double bond and Z is

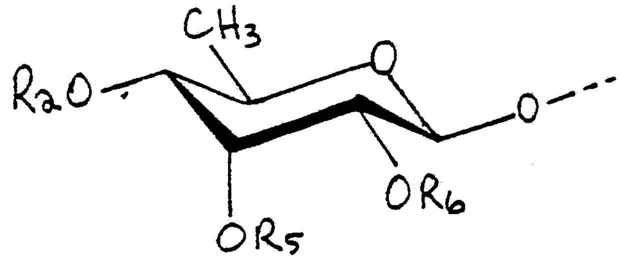

wherein $R_2$, $R_5$ and $R_6$ are as defined in claim 1.

6. A compound of claim 5 wherein $R_1$ is

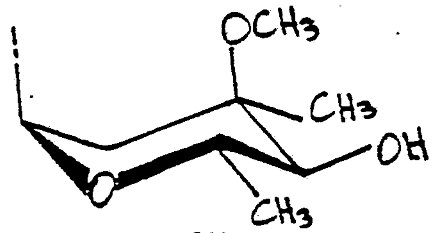

or

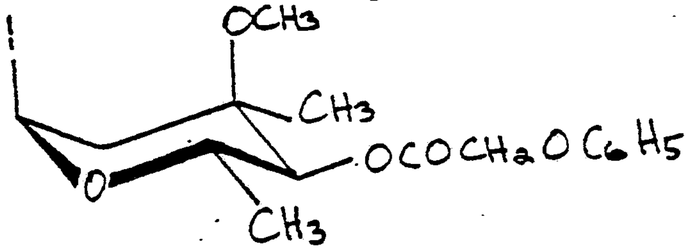

X is hydroxy or acetyl

Z is

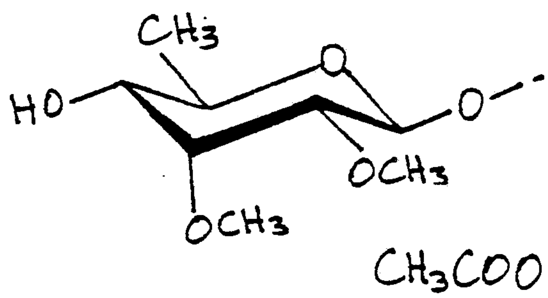

or

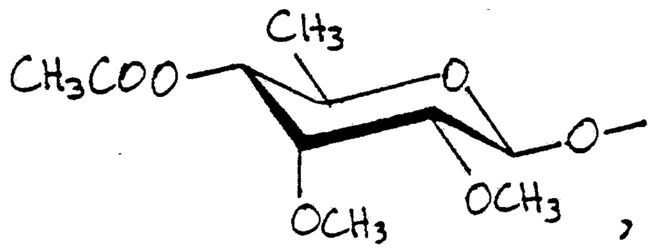

,

and $R_2$ is acetyl.

7. A compound of claim 1 which is 2″,4″,4‴, tri-O-acetyl-19-deformyl-O-(4′-O-phenoxyacetyl-α-L-cladinosyl)-(1→3)-desmycosin, or 2″,4″,4‴-tri-O-acetyl-O-(4′-O-phenoxyacetyl-α-L-cladinosyl)-(1→3)-desmycosin, or
2″,4″,4‴-tri-O-acetyl-20-deoxo-20-dihydro-O-(4′-O-phenoxyacetyl-α-L-cladinosyl)-(1→3)-desmycosin, or
O-(α-L-cladinosyl)-(1→3)-19-deformyldesmycosin, or
O-(α-L-cladinosyl)-(1→3)-20-deoxo-20-dihydrodesmycosin, or
O-α-L-cladinosyl-(1→3)-desmycosin.

8. A pharmaceutical composition comprising a compound of any one of claims 1 to 7 in admixture with a pharmaceutically acceptable carrier therefor.

9. The use of a compound of any one of claims 1 to 7 for the preparation of a pharmaceutical composition useful for treating bacterial infection.

10. A method for making a pharmaceutical composition comprising mixing a compound of any one of claims 1 to 7 with a pharmaceutically acceptable carrier.

European Patent Office

# EUROPEAN SEARCH REPORT

Application Number

EP 88 30 3869

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 97, no. 11, 13th September 1982, page 813, no. 92697m, Columbus, Ohio, US; & JP-A-82 05 000 (MICROBIOCHEMICAL RESEARCH FOUNDATION) 11-01-1982 * Abstract * | 1,3,8 | C 07 H 17/08<br>A 61 K 31/70 |
| X | CHEMICAL ABSTRACTS, vol. 102, no. 15, 15th April 1985, page 666, no. 132421x, Columbus, Ohio, US; & JP-A-59 128 400 (MICROBIOCHEMICAL RESEARCH FOUNDATION)24-07-1984 * Abstract * | 1,3 | |
| X,P | CHEMICAL ABSTRACTS, vol. 108, no. 19, 9th May 1988, page 693, no. 167885c, Columbus, Ohio, US; & JP-A-62 205 097 (TOYO JOZO CO., LTD) 09-09-1987 * Abstract * | 1-3,8 | |
| | | | TECHNICAL FIELDS SEARCHED (Int. Cl.4) |
| | | | C 07 H 17/00<br>A 61 K 31/00 |

The present search report has been drawn up for all claims

| Place of search | Date of completion of the search | Examiner |
|---|---|---|
| THE HAGUE | 22-08-1988 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document